Europäisches Patentamt

European Patent Office

Office européen des brevets

(11) Publication number: 0 271 201 A2

# EUROPEAN PATENT APPLICATION

(21) Application number: 87309680.4

(51) Int. Cl.4: C12N 15/00 , C12Q 1/70

(22) Date of filing: 02.11.87

The microorganisms have been deposited with the American Type Culture Collection under numbersATCC 67591 and ATCC 67592.

(30) Priority: 04.11.86 US 927278

(43) Date of publication of application:
15.06.88 Bulletin 88/24

(84) Designated Contracting States:
AT BE CH DE ES FR GB GR IT LI LU NL SE

(71) Applicant: SCRIPPS CLINIC AND RESEARCH FOUNDATION
10666 North Torrey Pines Road
La Jolla California 92037(US)

(72) Inventor: Nelson, Jay
3540 Stetson Avenue
San Diego California 92122(US)
Inventor: Oldstone, Michael B.A.
7710 Exchange Avenue
La Jolla California 92037(US)
Inventor: Southern, Peter
444 Marine
La Jolla California 92037(US)

(74) Representative: Fisher, Adrian John et al
CARPMAELS & RANSFORD 43 Bloomsbury Square
London WC1A 2RA(GB)

(54) Nucleic acid probes for the detection of latent human cytomegalovirus in blood products.

(57) Human cytomegalovirus (HCMV)-derived polynucleotide vectors and probes for detecting the presence of HCMV in a human cellular body sample are disclosed, as are diagnostic systems and methods for their use. The probes include a polynucleotide sequence that corresponds to an immediate-early protein gene or to a late protein gene operatively linked to an indicating means.

FIGURE 1

HCMV STRAIN AD169 DNA PROBES FOR *IN SITU* HYBRIDIZATION

-1-

# NUCLEIC ACID PROBES FOR THE DETECTION OF LATENT HUMAN CYTOMEGALOVIRUS IN BLOOD PRODUCTS

## Description

### Cross-Reference To Related Applications

This is a continuation-in-part of U.S. Patent Application Serial No. 726,454 filed on April 29, 1985, which itself is a continuation-in-part of U.S. Patent Application Serial No. 607,387 filed on May 4, 1984, the disclosures of which are hereby incorporated by reference.

### Technical Field

The present invention relates to the production of a human cytomegalovirus (HCMV) DNA probe and the use of such a probe to detect HCMV in a body sample. The invention is particularly useful in screening blood products for the presence of HCMV prior to transfusion.

### Background

#### A. Cytomegalovirus Infection

Cytomegalovirus inclusion disease (CID), generalized salivary gland virus disease, cytomegaly, and inclusion disease are synonyms for an illness caused by cytomegalovirus (CMV) infection. CMV is a species-specific agent with the physio-chemical and electron microscopic characteristics of a herpesvirus. Human cytomegalovirus (HCMV) was first isolated in fibroblastic tissue culture in the mid-1950s from infants with CID and from the adenoid tissue of school-age children. A cytopathic effect was noted in tissue culture which was characterized by large intranuclear inclusions. The propagation of the virus provided the basis for the development of specific serologic tests as will be described.

Cytomegalovirus infection is worldwide in distribution. Cytomegalovirus, however, does not

-2-

induce a highly communicable infection. Thus, a substantial number of individuals remain susceptible to the infecticn in adult life. CMV has been isolated from saliva, the upper respiratory tract, urine, milk, cervical secretions, semen, feces and circulating leukocytes in blood. The virus is probably transferred by intimate contact with an infected individual or by infusion of blood from a asymptomatic blood donor.

CMV is involved in a wide spectrum of clinical disorders from inapparent infection to severe congenital disease. Weller, T., N. Engl. J. Med., 285, 203 and 267 (1971).

For example, disease occurs in patients on immunosuppressive therapy and in those subject to opportunistic infections. In fact, CMV has become the most common infection after allogeneic bone marrow transplantation and is an important determinant of the success or failure of the transplant procedure [Neiman et al., J. Infect. Dis., 136, 754 (1977)].

Among adults undergoing immunosuppressive therapy after renal homotransplantation, over 90 per cent develop an active cytomegalovirus infection if they have cytomegalovirus antibody prior to surgery. Approximately half of the seronegative patients subsequently become infected on immunosuppressive therapy. Seronegative recipients receiving a kidney from a seropositive donor almost always develop a postoperative infection and are likely to develop symptoms.

HCMV infection has also been reported to cause encephalitis, occurring more frequently in immunosuppressed patients [Dorfman, Neurology, 23, 136 (1974): Kauffman et al., Am. J. Med., 67, 724

-3-

(1979); Schober et al., <u>Lancet.</u> <u>1</u>, 962 (1973). Documentation of this diagnosis of HCMV infection has been hindered by the difficulty of recovering virus from cerebrospinal fluid (CSF) or brain tissue and has relied instead on histological examination of infected tissues.

Examples of HCMV encephalitis range from cases involving immunosuppressed patients after receiving organ transplants [Bale, <u>Arch. Neurol.</u>, <u>41</u>, 310 (1984)] including kidney [Schneck, <u>J. Neuropathol. Exp. Neurol.</u>, <u>24</u>, 415 (1965)] to patients afflicted with acquired immune deficiency syndrome (AIDS) [Levy et al., <u>J. Neurosurgery</u>, 61, 9 (1984); Moskowitz et al., <u>Arch. Pathol. Lab. Med.</u>, <u>108</u>, 867 (1984); Nielsen et al., <u>Am. J. Clin. Pathol.</u>, <u>82</u>, 678 (1984); Snider et al., <u>Ann. Neurol.</u>, <u>14</u>, 403 (1984)].

Specifically, a type of cytomegalovirus infection has been described in patients who have received blood transfusions. This condition, referred to as post-transfusion CMV mononucleosis, occurs two to four weeks after the administration of blood.

Acquired cytomegalovirus infection has also been associated with autoimmune hemolytic disease, ulcerative lesions of the gastrointestinal tract, post-transplantation pneumonia and thrombocytopenic purpura. For the majority of CMV infections acquired after birth, however, recovery is without significant complications.

There has been indirect evidence for an important role for lymphocytes in human cytomegalovirus (HCMV) infection. For example, individuals with HCMV mononucleosis have atypical lymphocytes, and <u>in vitro</u> tests reveal depressed

-4-

lymphocyte functions. [Rinaldo et al., J. Infect. Dis., 136, 667 (1977); Carney et al., J. Infect. Dis., 144, 47 (1981); and Rinaldo et al., J. Infect. Dis., 141 (1980)]. HCMV infections can be transferred from healthy donors [Stevens et al., J. Am. Med. Assoc., 221, 1341 (1970); Armstrong et al., Transfusion, 3, 159 (1971); Lang et al., N. Engl. J. Med., 278, 1147 (1968); Lang et al., Ibid, 280, 1149 (1969); Prince et al., Ibid., 284, 1125 (1971); and Lang et al., Transfusion (Philadelphia), 17. 391 (1977)] (70 percent of adults are seropositive for HCMV) by transfusion. However, it has only been recently shown that a small percentage (1-5 percent) of lymphocytes infected in vitro with HCMV express immediate-early and early proteins [Rice et al., Proc. Natl. Acad. Sci. USA, 81, 6134 (1984)], and that a latent CMV infection in lymphocytes can be demonstrated by protein expression and RNA detection [Schrier et al., Science, 230, 1048 (Nov. 1985)].

Past attempts to define the infection of lymphocytes with HCMV have been hampered by several factors including use of long-term laboratory strains of HCMV which appear to be less infective than fresh clinical isolates [Rice et al., Proc. Natl. Acad. Sci. USA, 81, 6134 (1984)], the cryptic nature of the infection [J. Pagano, J. Infect. Dis., 132, 209 (1975)], and the lack of sensitive methods to detect low levels of virus in a small percentage of cells. Moreover, a characteristic of herpes viruses is the ability of the virus to persist in the host tissue for many years after initial infection and later be reactivated to cause disease, e.g., HSV-1, HSV-2 and varicella zoster virus (VZV) in sensory ganglia and EBV in B lymphocytes. Although CMV appears to cause a lifelong infection, the site of the latent infection is unknown. In the murine CMV model, virus can be recovered from activated, B cells so there are indications that lymphocytes may not only be involved in the disease state and transmission, but also could be a site of virus persistence.

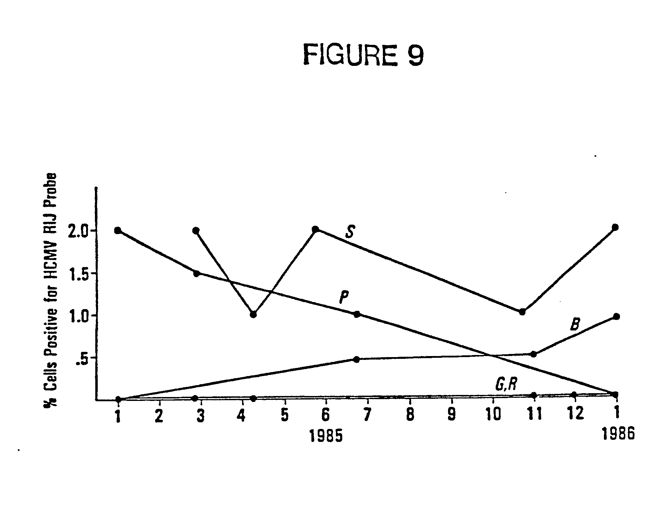

The diagnosis of cytomegalovirus infection in a patient with mononucleosis-like symptoms can be established by virus isolation from infected tissues. Antibodies produced in response to infection with a CMV can be detected by neutralization (NT), complement-fixation (CF), immunofluorescence and platelet-agglutination (PA) procedures. [See Weller, T. H., N. Engl. J. Med., 285, 203 (1971)]. Currently available serologic tests, however, must be interpreted with caution when diagnosing CMV infection because of cross-reactions with other cell-associated herpes viruses, and, for example, the tendency of CF antibody to fluctuate widely in normal individuals. In addition, as is shown in Figure 9, some HCMV seropositive individuals do not show detectable virus in the blood.

There is no satisfactory treatment for cytomegalovirus infections. The use of corticosteroids, gamma globulin and antiviral drugs such as deoxyuridine, floxuridine, cytosine arabinoside and adenine arabinoside have been reported with equivocal, beneficial, or no effect on the clinical course. But these reports are difficult to evaluate because of the small numbers of individuals treated, the multiple factors operating simultaneously and the variability of virus excretion in individuals tested at different times.

B.  The HCMV Genome

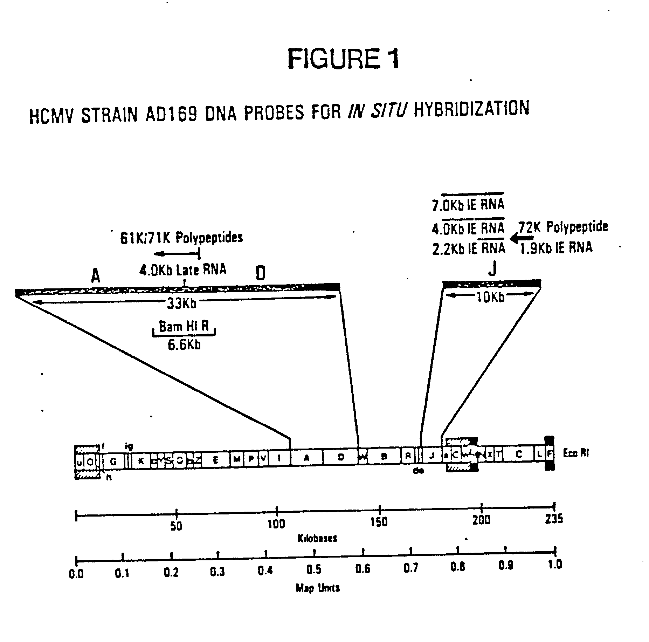

The genome of HCMV strain AD169 is a linear, double stranded DNA molecule with a size of

-6-

approximately 235 kbp (kilobase pairs). See Figure 1 herein; Weststrate et al., J. Gen. Virol., 49, 1 (1980) and Fleckenstein et al., Gene, 18, 39 (1982,.. This molecule contains two unique stretches of DNA denominated $U_L$ and $U_S$ that are bordered by a pair of inverted repeats. Weststrate et al., J. Gen. Virol., 49, 1 (1980). The DNAs of different strains of HCMV demonstrate 80 percent homology by reassociation kinetics. Huang et al., Yale J. Biol. Med., 49, 29 (1976). However, HCMV DNA has less than 5 percent homology with herpes simplex virus types 1 and 2 (HSV-1 and HSV-2, respectively) and murine and simian cytomegaloviruses. Huang et al., J. Virol., 13, 642 (1974).

Transcription of the major laboratory strains (Davis, Towne and AD169) can be divided into three separate phases. DeMarchi, Virology, 114, 23 (1981); Wathen et al., J. Virol., 41, 462 (1982) and McDonough et al., Virology, 125, 31 (1983). The RNA species transcribed prior to protein synthesis have been designated immediate-early (IE) RNAs and are transcribed in restricted areas of the genome. DeMarchi, Virology, 114, 23 (1981); and Wathen et al., J. Virol., 41, 462 (1982). After the onset of viral protein synthesis, another class of RNA species, early RNA, is transcribed throughout the genome but primarily on the inverted terminal repeats. Wathen et al., J. Virol., 41, 462 (1982). The late RNAs are transcribed after the onset of viral DNA replication.

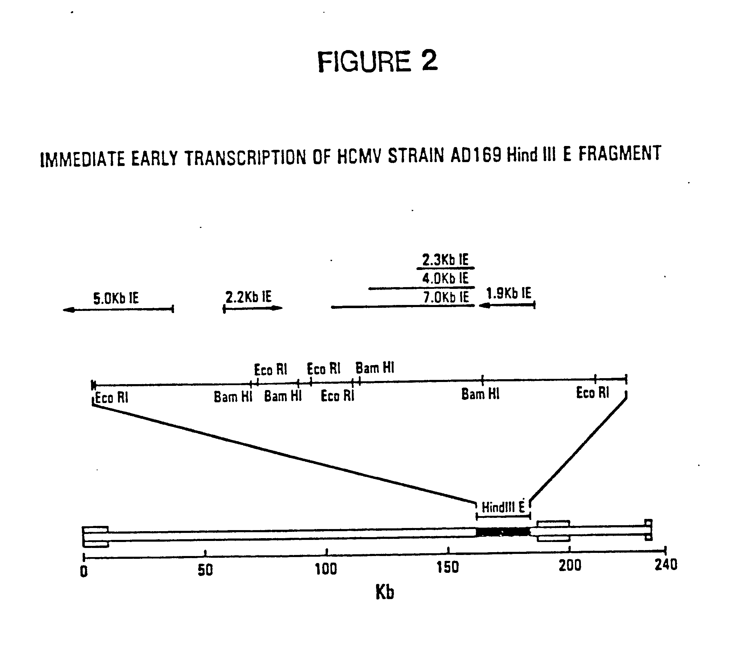

The HindIII E fragment of HCMV strain AD169 [map units of about 0.06 to about 0.16, Nelson et al., J. Virol., 43, 83 (1982)] contains 90 percent of the coding region for transcription of immediate-early RNAs. Wathen et al., J. Virol., 41, 462 (1982) and McDonough et al., Virology, 41, 462 (1982). Four abundantly transcribed IE RNAs (1.9 kb, 2.2 kb, 2.3 kb and 5.0 kb) and two minor transcripts are produced in this region. Jahn et al., J. Virol., 49, 363 (1984). (See Figure 2).

One of the most characterized genes in the HCMV genome is the major IE gene. This transcription unit produces a 1.9 kb RNA located between a SstI and BamHI site located on the HindIII E fragment of the AD169 genome. [Nelson et al., Mol. Cell. Biol., 6 452 (1986)]. This messenger RNA (mRNA) is the most abundant transcript in the IE region and codes for the major IE 72,000 dalton (72 K) protein [as reported by Stinski et al., J. Virol., 46, 1 (1983)] that can be detected by the monoclonal antibody L-14. Rice et al., Proc. Natl. Acad. Sci. USA, 81, 6134 (1984).

Monoclonal antibody (mab) L-14 reacts specifically with CMV-infected cells. In particular, mab L-14 reacts with a protein that appears early after CMV infection (within 3 hours) and remains localized to the cell nucleus throughout the infectious cycle. The hybridoma that produces mab L-14 was deposited on May 3, 1984 at the American Type Culture Collection, Rockville, Maryland and was given the ATCC accession number HB 8554.

Recently, a structural analysis of this 1.9 kb mRNA from the Towne strain indicated that the transcript was a spliced molecule containing 4 exons of 1341, 185, 88 and 121 nucleotides, respectively. Stenberg et al., J. Virol., 49, 190 (1984). This IE protein is preferentially associated with chromatin, and it has been proposed that this protein plays a major role in influencing transcription of other viral genes. Stinski et al., J. Virol., 46, 1

-8-

(1983). Rat-2 cells microinjected with clones encoding this IE gene were shown to rapidly express (within about 2 hours) the 72 K protein by immunofluorescence using the E-3 monoclonal antibody. Goldstein et al., Infect. and Immun., 38, 273 (1982).

These results suggest that even though HCMV has a restricted host range, expression of the major IE gene is not dependent on replication of the virus. However, during a permissive infection, transcription of the 1.9 kb IE RNA is not detectable at 48 hours post-infection demonstrating temporal regulation of the gene. Nelson et al., Mol. Cell. Biol. 6, 452 (1986).

C. Viral Diagnostic Methods

Conventional viral diagnostic methods typically consist of viral isolation and serology studies. When sensitive cell cultures are used, certain common viruses like herpes simplex, enteroviruses and influenza viruses can be isolated within 1 to 4 days. However, other viruses including cytomegalovirus may require 7 to 14 days or longer for isolation. Thus, while viral isolation remains the standard against which other viral diagnostic techniques are measured, the development of more rapid methods is imperative.

An ideal rapid diagnostic method would involve the direct examination of clinical specimens with a result obtained within hours of collection. Electron microscopy has been used for rapid viral diagnosis, but it is too expensive, time-consuming and insensitive to be used for the routine screening of large numbers of specimens. For many years, rapid viral diagnostic methods centered around viral antigen detection and included immunofluorescence,

-9-

radioimmunoassay, immunoperoxidase and more recently, enzyme-linked immunosorbent assay. These methods have been applied directly to clinical specimens, to biopsy and autopsy tissues and to cell cultures after viral inoculation and amplification in order to obtain more rapid results. In general, however, these methods have not been as sensitive as viral isolation when applied directly to clinical specimens.

A variety of polynucleic acid (polynucleotide) hybridization techniques have been developed over the years to investigate the structure and expression of cellular and viral genes. In the past few years, polynucleic acid hybridization technology has been applied to clinical virology, both for rapid diagnosis using clinical specimens and for studies of viral pathogenesis.

The advantages of polynucleic acid hybridization include its specificity in that labeled polynucleic acid probes anneal to complementary polynucleotide sequences in a test sample, and its sensitivity, based on the strong avidity of complementary sequences for each other. Hybridization can also be used, as described herein, to detect nonreplicating viral infections that are abortive or latent.

The DNA molecule in its natural state is made up of two strands wound helically around each other, with each base specifically linked by hydrogen bonds to a complementary base on the other strand. For example, thymine is always linked by hydrogen bonds to adenine, and guanine is always linked by hydrogen bonds to cytosine. The hydrogen bonds between the bases can be broken by heating so that the two strands of DNA are completely separated from each other. The dissociation of DNA (also called denaturation or melting) occurs at a temperature that is characteristic for each DNA. The melting temperature ($T_m$) of each DNA varies according to the guanine plus cytosine content and the length of the DNA, the ionic strength of the solution, and the presence of organic solvents such as formamide. For example, each 1 percent formamide in the reaction mixture generally reduces the $T_m$ by about 0.7 degrees Centigrade (C).

When left in solution at a temperature below the $T_m$, the dissociated single polynucleic acid strands collide with complementary partners, and if the conditions are right, double-stranded helixes are again formed. The rate of reassociation is maximal at about 25 degrees C below the $T_m$ for that DNA.

Reassociation or annealing of a polynucleotide is primarily determined by the polynucleotide concentration and the time of incubation. Other factors that affect the rate of reassociation are the complexity of the polynucleotide (the more complex, the slower the rate), the ionic strength of the solution (increasing ionic strength increases the rate of reassociation), and the presence of high molecular weight polymers such as dextran sulfate, which can increase the rate of reassociation from 3 to 100 times.

The stability of reassociated double-stranded polynucleotide depends on the degree to which the two strands are composed of complementary base sequences, which in turn determines how well the two strands are hydrogen bonded. When hybridization proceeds under stringent conditions, only highly complementary strands will anneal. Under conditions of low stringency, the

-11-

formation of hybrids with some degree of base mismatching can occur. Conditions of low stringency can be useful when it is advantageous to detect related, but not identical, polynucleic acid sequences. This has been done with human papillomaviruses, herpes simplex virus types 1 and 2, adenoviruses, enteroviruses and rotaviruses.

In standard procedures, a labeled single-stranded polynucleic acid "probe" containing the specific sequence or sequences complementary to the sequence(s) being sought is mixed with a denatured (dissociated) sample of DNA or RNA. Under appropriate conditions, the labeled probe anneals with complementary nucleic acid sequences in the sample, forming double-stranded "hybrids," which now contain the label. By several known methods, single- and double-stranded polynucleic acids can be separated and the label can be quantitiated.

Brief Summary of the Invention

The present invention contemplates a polynucleotide probe to human cytomegalovirus (HCMV), to a process for preparing a probe, and to the use of such a probe to assay for the presence of HCMV in a human body sample.

One aspect of this invention relates to a method for detecting latent human cytomegalovirus in a human body sample. That process comprises the steps of: (a) admixing a sample of fixed, permeablized human cellular body sample such as peripheral blood mononuclear cells (PBMs) like lymphocytes with a recombinant HCMV-derived polynucleotide probe whose sequence corresponds to either or both of genes expressed as an immediate-early or late RNA transcript by the virus in an amount sufficient to anneal any complementary

-12-

polynucleotide present; i.e., the fixed, permeablized cells are admixed with a polynucleotide of this invention that includes an indicating means; (b) maintaining the admixture under hybridization conditions for a time period sufficient for annealing of the probe to any complementary polynucleotide present in the sample to occur; (c) separating the complexed probe and complementary sequence from any uncomplexed probe; and (d) assaying for the presence of a signal from the indicating means.

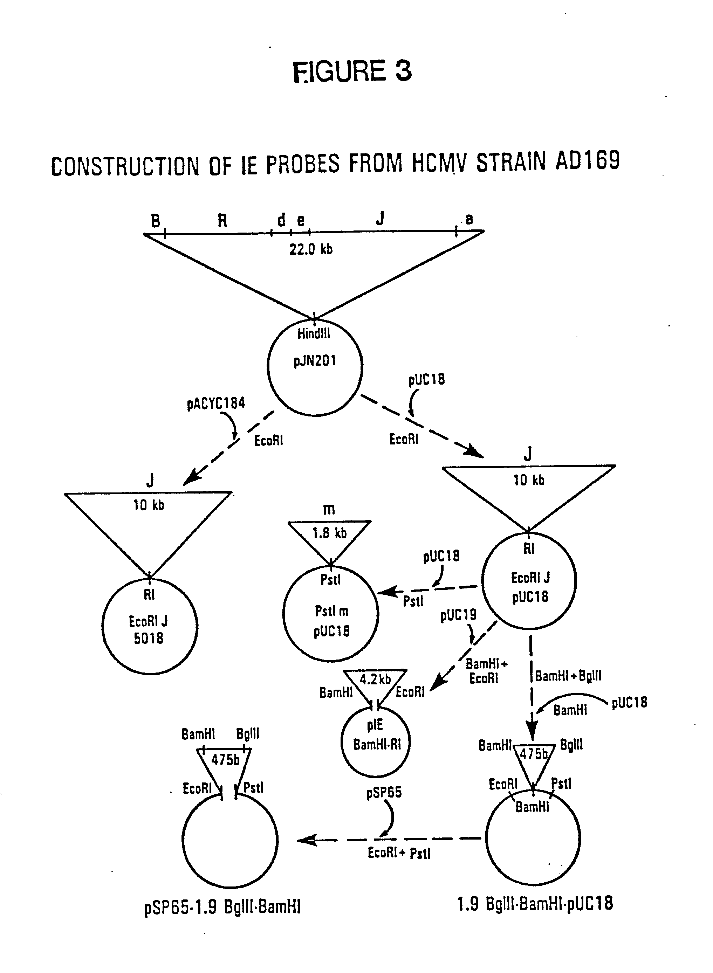

Another aspect of the present invention relates to a process for preparing a recombinant HCMV-derived plasmid or probe. That process comprises the steps of: (a) digesting the HindIII E fragment of an HCMV strain DNA with restriction endonuclease EcoRI to provide fragmented linear DNA segments including an EcoRI J fragment of about 10 kb, as shown in Figure 3; (b) ligating the EcoRI J fragment into the EcoRI site of a plasmid to provide a recombinant HCMV-derived plasmid; (c) cloning the recombinant plasmid in a suitable replication medium such as a bacterium to provide an amount of the recombinant plasmid sufficient for use as a probe; and (d) linking an indicating means to the plasmid or the EcoRI J fragment thereof to form the probe. The recombinant plasmid so formed is thus cloned (replicated) in a replication medium to produce a greater amount of that plasmid, the cloned, recombinant HCMV-derived plasmid or EcoRI J fragment is recovered from the replication medium and linked to the indicating means for use as a DNA probe.

In another embodiment, this invention includes a process for preparing a recombinant HCMV-derived plasmid or probe comprising the steps of digesting the before-described EcoRI J

-13-

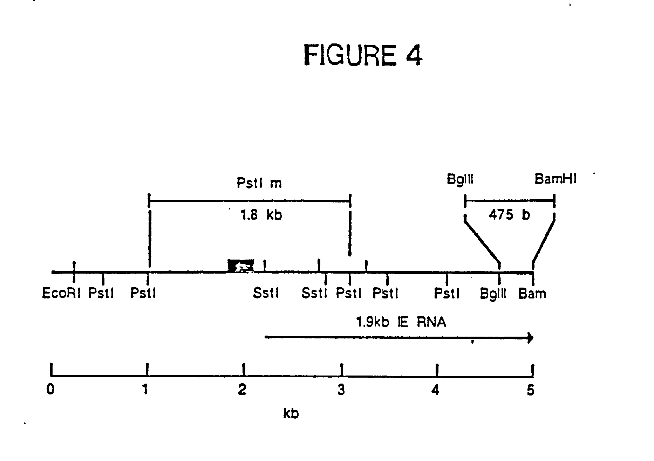

fragment-containing recombinant HCMV-derived plasmid with restriction endonuclease PstI to provide fragmented linear DNA segments including a PstI m fragment, of about 1.8 kb, as shown in Figure 4 and ligating the PstI m fragment into the PstI site of a second plasmid to provide a second recombinant HCMV-derived plasmid. That second plasmid is typically cloned in a suitable replication medium to provide more of the plasmid, and an indicating means is linked to the resulting plasmid or at least to the PstI m in fragment portion thereof to form the desired probe.

In still another embodiment of this invention, a foregoing process can further include the additional steps of: digesting the first-named recombinant HCMV-derived EcoRI J fragment-containing plasmid with restriction endonucleases BglII and BamHI to provide fragmented linear DNA segments including a BglII-BamHI fragment of about 475 bases as shown in Figure 4; and ligating the BglII-BamHI fragment into the BamHI site of the polylinker of plasmid pUC18 to provide a third recombinant HCMV-derived plasmid. An indicating means is linked to the plasmid or to at least the BglII-BamHI portion thereof to form the probe.

An RNA probe is prepared by digesting the above-described third recombinant HCMV-derived plasmid with Eco-RI and PstI endonucleases to provide an EcoRI-PstI fragment as shown in Figure 3 that includes the before-mentioned BglII-BamHI fragment of about 475 bases. That EcoRI-PstI fragment is then ligated into the respective sites of an in vitro transcription plasmid such as pSP65 to form a fourth plasmid. The fourth plasmid is thereafter cloned in a suitable replication medium to prepare more of it.

0 271 201

-14-

The resulting plasmid is thereafter linearized with an appropriate endonuclease, and an RNA probe is prepared from the resulting linearized plasmid by an *in vitro* run-off transcription reaction carried out in the presence of one or more appropriately labeled ribonucleic acid triphosphates.

A further embodiment of this invention includes a process for preparing a recombinant HCMV-derived plasmid or probe comprising the steps of: (a) digesting the first-named recombinant HCMV-derived EcoRI J fragment-containing plasmid with restriction endonucleases BamHI and EcoRI to provide fragmented linear DNA segments including a BamHI-EcoRI fragment of about 4.2 kb as shown in Figure 3; and (b) ligating the BamHI and EcoRI sites of the polylinker of plasmid pUC19 to provide a fifth recombinant HCMV-derived plasmid. That fifth plasmid is typically cloned in a suitable replication medium to provide more of the plasmid, and an indicating means is linked to the resulting plasmid or at least to the BamHI-EcoRI fragment portion thereof to form the desired probe.

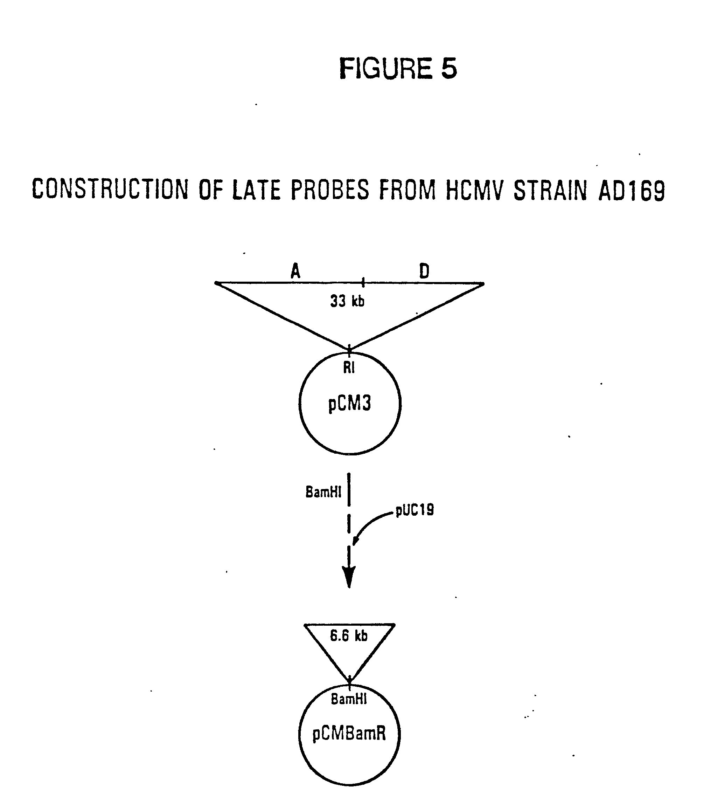

In yet another embodiment of this invention, a process is contemplated for preparing a recombinant HCMV-derived plasmid and probe related to a late gene protein product comprising the steps of: (a) digesting the EcoRI A plus D (A-D) fragment of an HCMV strain DNA with restriction endonuclease BamHI R fragment of about 6.6 kb as shown in Figure 5; and (b) ligating the BamHI R fragment into the BamHI site of the polylinker of plasmid pUC19 to provide a sixth recombinant HCMV-derived plasmid. That sixth plasmid is typically cloned in a suitable replication medium to provide more of the plasmid, and an indicating means is linked to the resulting plasmid or at least to the BamHI R fragment portion thereof to form the desired probe.

In any of the embodiments described herein, the HCMV strain can comprise one of the frequently used HCMV strains including Davis, Towne and AD169; and the plasmids can be selected from pACYC184, pUC18, pUC19, the so-called Riboprobe vector pSP65 and other plasmids or cosmids well known to those skilled in the art.

Each of the before-described HCMV-derived plasmids is preferably cloned in a replication medium such as a unicellular organism like E. coli to produce a greater amount of the plasmid in the medium. The cloned HCMV-derived plasmid is thereafter recovered from the replication medium.

For use as DNA probes, the cloned, recovered plasmids are operatively linked to an indicating means, as noted, that is capable of indicating the presence of the probe annealed to HCMV nucleic acid in cells to be assayed. Linkage of an indicating means to the plasmid to form the DNA probe can be carried out in a number of manners, but nick-translation is the preferred method.

Other aspects of this invention include the recombinant HCMV-derived plasmids and probes. Starting preferred recombinant HCMV-derived plasmids (PJN201 and pCM3) useful in preparing plasmids and probes of this invention have been deposited at the American Type Culture Collection (ATCC) in Rockville, Maryland and have been assigned the following ATCC accession numbers _____ and _____, respectively. Those plasmids were contained in E. coli and were deposited on _____ pursuant to the Budapest Treaty.

0 271 201

-16-

A further aspect of this invention is a diagnostic assay system for detecting the presence of HCMV in a biological sample. Such an assay system is particularly useful in screening blood products for latent HCMV prior to transfusion of one or more blood constituents.

The system comprises at least one container, and includes as an active ingredient, an amount of an indicating means-linked recombinant HCMV-derived probe of this invention corresponding in sequence to an immediate-early protein gene sufficient to react with and anneal to complementary nucleic acid sequences in the sample.

An immediate-early gene-directed probe is particularly useful for detecting a latent HCMV infection. A combination of probes, one directed to the immediate-early gene of the 72 K protein product and one directed to the gene of a late protein-product, is preferred for detecting infection in a patient where it is not known whether infection is at the stage of transcribing immediate-early genes or has progressed to a stage of transcription in which the late genes but not immediate-early genes are being expressed. Thus, another diagnostic system comprises at least two containers, one of which includes the above active ingredient and the other of which includes as an active ingredient, an amount of an indicating means-linked HCMV-derived probe of this invention directed to a late gene protein product sufficient to react with and anneal to complementary nucleic acid sequences in the sample.

The indicating means can include radioisotopes such as $^3$H, $^{32}$P, $^{14}$C and $^{35}$S.

Another particularly useful indicating means is biotin which can produce a biotin-labeled probe that is stable for up to two years when stored at -20 degrees C. Biotin-containing probes are typically used with avidin-containing amplifying and visualizing reagents.

Brief Description of the Drawings

In the Figures which comprise a portion of this disclosure:

Figure 1 is a physical map of the EcoRI fragments of HCMV DNA, strain AD169, showing the relative positions of the viral DNA fragment sequences used herein for in situ hybridization, as well as the abundantly transcribed RNAs produced by the EcoRI A, D and J fragments.

Also illustrated in the lower portion of the Figure is a composite map that illustrates the approximate positions of the EcoRI restriction endonuclease cleavage sites and the letter designations of the fragments produced by those cleavages. This map is shown in the opposite direction from that taken from Fleckstein et al., Gene, 18, 39 (1982), at page 44.

Figure 2 is a map of the HindIII E DNA fragment of HCMV strain AD169, illustrated from left to right and in the direction of the 5'-end to the 3'-end, that includes the coding region for immediate-early RNAs that are transcribed before protein synthesis. Approximate positions of the EcoRI and BamHI restriction endonuclease cleavage sites within the HindIII E fragment are illustrated, as are the approximate positions of the six RNA transcripts from this region.

Figure 3 is a schematic diagram showing the subcloning of various segments of the HCMV EcoRI J fragment contained within the HindIII E fragment present in the recombinant plasmid pJN201 described by Nelson et al., J. Virol., 43:83-91 (1982). In this scehmatic, the 10 kb EcoRI J fragment is isolated from pJN201 and cloned into the vector pACYC184 to form the plasmid identified as EcoRI J-5018. Also shown is the sequential cloning of EcoRI J fragment into the vector pUC18 to provide a first recombinant HCMV-derived plasmid EcoRI J-pUC18; the cloning of a PstI m fragment into the vector pUC18 to provide a second recombinant HCMV-derived plasmid PstI m-pUC18; the cloning of a BglII-BamHI fragment into the vector pUC18 to provide a third recombinant HCMV-derived plasmid 1.9 BglII-BamHI-pUC18; the cloning of an EcoRI-PstI fragment that contains the BglII-BamHI fragment into the Riboprobe vector pSP65 to provide a fourth recombinant HCMV-derived plasmid denominated 1.9 BglII-BamHI-pSP65; and the cloning of the BamHI-EcoRI fragment located on the 5'-end of the EcoRI J fragment relative to the 1.9 kb IE RNA transcript into the vector pUC19 to provide a fifth recombinant HCMV-derived plasmid pIEBamHI-RI.

Figure 4 is a restriction endonuclease map, illustrated from left to right and in the direction of the 5'-end to the 3'-end, showing the coding region for the EcoRI J DNA fragment of the HindIII E fragment of HCMV strain AD169. Approximate positions of the PstI, SstI, BglII and BamHI restriction endonuclease cleavage sites within the EcoRI J fragment are illustrated as are the approximate positions of the PstI m fragment and the BglII-BamHI fragment.

Figure 5 is a schematic diagram showing the cloning of a HCMV 6.6 kb BamHI fragment denominated BamHI R into the BamHI site of the plasmid vector pUC19 to form the recombinant plasmid pCMBamR. The BamHI R fragment is derived from the plasmid vector pCM3 which contains the combined HCMV 33 kb EcoRI A plus D (A-D) fragments cloned into cosmid pHC79 [Fleckenstein et al., Gene 18, 39 (1982)] by reaction with BamHI to provide fragmented linear DNA segments including the 6.6 kb BamHI R fragment. The recombinant plasmid, pCMBamR, when linked to an indicating means is useful as a DNA probe for the 4.0 kb late HCMV RNA.

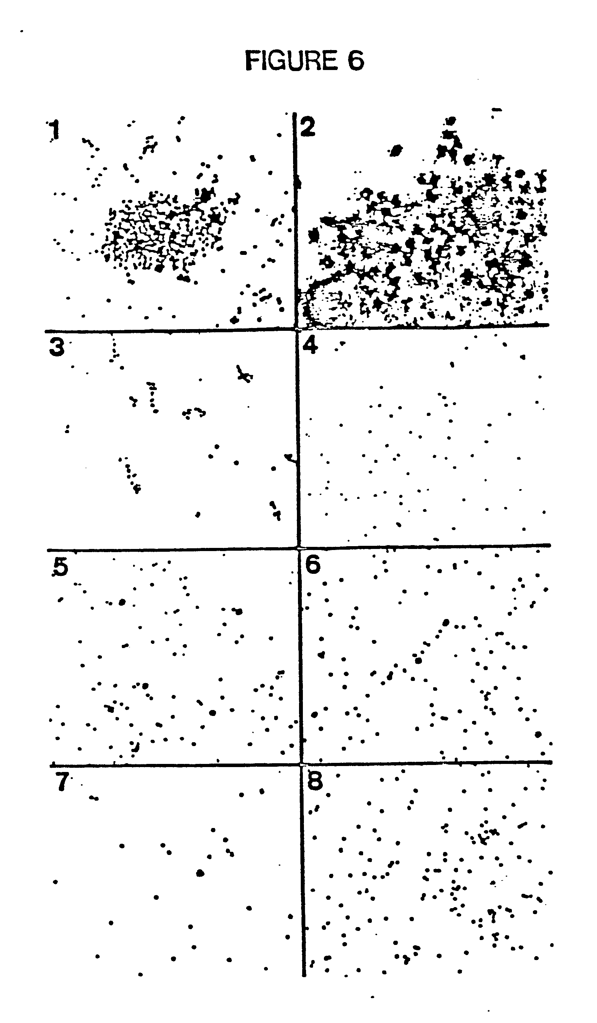

Figure 6 contains a composite image from eight photomicrographs, that are labeled 1-8. Panel 1 shows in situ hybridization of peripheral blood mononuclear cells (PBM) from HCMV-seropositive donor A using an $^{35}$S-labeled probe denominated pUC 18-EcoRI J at a magnification of 20x. Panel 2 illustrates a portion of panel 1 at a magnification of 100x. Panel 3 shows the results of an in situ hybridization study using the above pUC18-EcoRI J probe and PBM from donor A that were treated with 0.1 M NaOH for a period of 30 minutes at 37°C prior to hybridization. Panel 4 shows PBM from an HCMV-seronegative donor hybridized with probe pUC18-EcoRI J. Panel 5 is similar to panel 1 except that PBM were from HCMV-seropositive donor B. Panel 6 shows cells from donor B that were preselected by FACS for the OKT4 marker and then hybridized as in panel 5. Panel 7 shows cells from donor B that were preselected by FACS for the OKT8 marker and then hybridized with the $^{35}$S-labeled pUC18-EcoRI J probe. Panel 8 shows PBM from donor B hybridized with $^{35}$S-labeled pUC18.

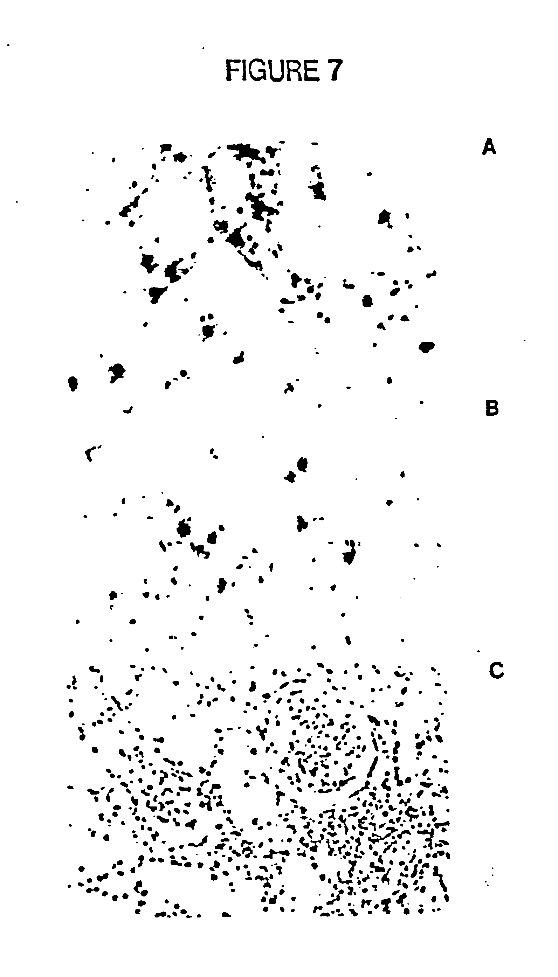

Figure 7 contains a composite image from three photomicrographs demonstrating in situ hybridization to thin-sections of HCMV-infected kidney using HCMV immediate-early or late gene probes.

Thin sections of an HCMV-infected patient's kidney were obtained, formalin fixed, paraffin-embedded and subjected to in situ hybridization using $^{35}$S-labeled HCMV-derived probes and counterstained with hematoxylin as described in the Materials and Methods Section. Frame A shows hybridizaion using the immediate-early HCMV gene-derived probe, EcoRI J (400x); Frame B shows hybridization using the late HCMV gene-derived probe, pCM3 (400x); and Frame C shows no hybridization when using a negative control probe, pUC18 (250x).

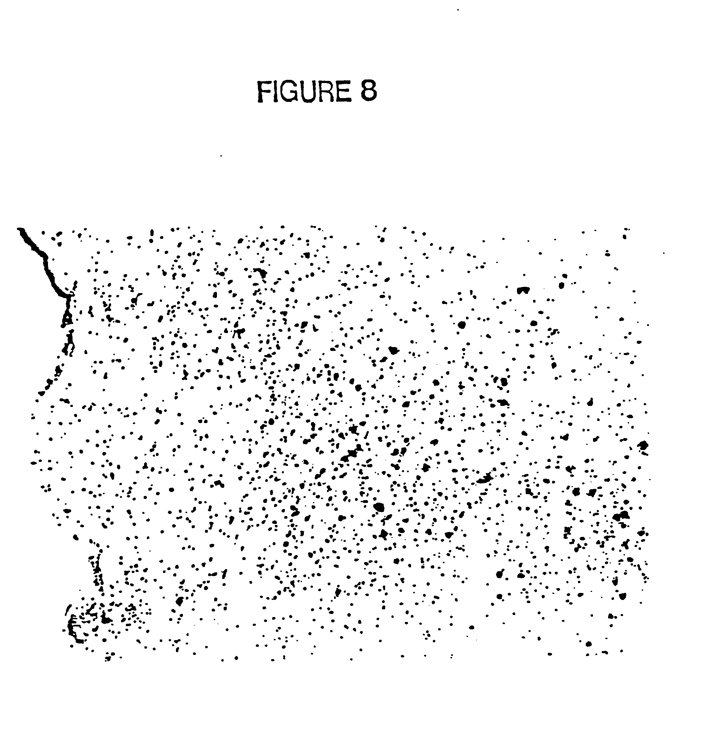

Figure 8 contains a photomicrograph showing in situ hybridization to thin-sections of HCMV-infected human brain using HCMV immediate-early gene probes. Paraffin-embedded sections of periventricular tissue were in situ hybridized with the $^{35}$S-labeled HCMV immediate-early gene probe, EcoRI J, and counterstained with hematoxylin, as described hereinafter (400x).

Figure 9 is a graph that illustrates fluctuations in hybridization to PBMs from normal HCMV serpositive donors (G, R, B, P and S) using the immediate-early EcoRI J probe as described in the Materials and Methods Section. Repeat analysis over a one year period by in situ hybridization of PBMs from asymptomatic normal seropositive donors showed that the hybridization levels can either be stable for a given individual or can vary over time. The ordinate is in units of the percentage of cells that exhibited positive hybridization with the probe, whereas the abscissa illustrates the months of 1985 and 1986 in which the PBMs were obtained from the donors.

-21-

## Detailed Description of the Invention

### I. DEFINITIONS

The polynucleic acid (polynucleotide) probes useful herein are utilized along with an "indicator labeling means", "indicating means", "indicating group" or a "label", all of which terms are used interchangeably. The indicating group or label is utilized in conjunction with the probe as a means for signalling (determining) that a complementary HCMV DNA or RNA has bound to the probe.

The above terms are used herein to include single atoms and molecules that are linked to the probe whether those atoms or molecules are used alone or in conjunction with additional reagents. Such indicating groups or labels are themselves well-known in biochemistry and constitute a part of this invention only insofar as they are utilized with otherwise novel probes, methods and/or systems.

The signal-providing label utilized is operatively linked to the probe, as discussed hereinafter.

The term "operationally linked" is used herein to mean that one entity is linked to another entity such that the normal function of both entities is not substantially impaired. Thus, where a label is operationally linked to a polynucleotide in a probe, the polynucleotide functions to bind to a complementary polynucleotide strand and the indicating means functions to indicate that pairing and annealing of the probe strand and complementary strand has occurred. Where a polynucleotide strand is operationally linked into a vector by ligation, the resulting recombinant vector is capable of replication. Whenever one entity is described herein as being linked to another entity, it is to be understood that those entities are operatively linked whether the word "operatively" is used or not.

The indicating group can be or utilize a biologically active enzyme, such as horseradish peroxidase (HRP) or glucose oxidase, or the like. Where the principal indicating group is an enzyme such as HRP or glucose oxidase, additional reagents are required to visualize the fact that a complex has formed betweem the probe and a complementary strand of nucleic acid. Such additional reagents for HRP include hydrogen peroxide and an oxidation dye precursor such as diaminobenzidine. An additional reagent useful with glucose oxidase is 2,2'azino-di-(3-ethylbenzthiazoline-6-sulfonic acid) (ABTS).

Radioactive elements provide another, particularly preferred class of label, and are used herein as exemplary of useful labels. An exemplary radiolabeling agent that can be utilized in the invention is a radioactive element that produces beta ray emissions. Elements which themselves emit beta rays, such as $^3H$, $^{14}C$, $^{32}P$ and $^{35}S$ represent a class of beta ray emission-producing radioactive element indicating groups.

The term "probe" is used herein to mean a polynucleotide sequence of DNA or RNA that contains a linked indicating means, as discussed above, that is complementary to a messenger RNA or to a single strand of DNA that encodes a protein such as an immediate-early or late protein expressed by HCMV. A probe is utilized as a single strand of polynucleic acid, but for convenience is sometimes referred to herein as the double-stranded molecule such as an indicator means-linked plasmid. As is discussed elsewhere herein, the single-stranded probe is

-23-

typically prepared from the double-stranded molecule by heating and rapid cooling prior to or after admixture of the probe with the biological sample to be assayed.

A probe described herein is utilized in a polynucleotide hybridization study in which a single-stranded probe anneals to a complementary strand of another polynucleotide within a cell. Such hybridization studies are well known in the art as in situ hybridization studies. In such hybridization procedures, both the probe and its soughtafter complementary polynucleotide sequence must be single-stranded, as already noted.

Where the probe contains DNA molecules, it must be melted to form single-stranded molecules. Where the complementary soughtafter polynucleotide is also a DNA molecule, that molecule must also be melted before the probe can anneal to it. Thus, where a DNA probe is used to hybridize to a soughtafter DNA, both double-stranded molecules must be melted at some time during the study so that the desired annealing can occur. Those melting steps can be carried out independently, or after both double-stranded molecules are admixed.

Where a DNA probe is used to seek RNA as is discussed hereinafter, only the DNA probe need be melted for use. However, the double-stranded DNA probe and RNA within the cell can also be melted after admixture, if desired. No melting is needed wherein an RNA probe is utilized to seek a complementary RNA since RNA probes are single-stranded as is a soughtafter RNA.

In addition, while polynucleotides sought in Southern or Northern hybridization blots are relatively available to the probe, polynucleotides sought in an _in situ_ hybridization study are relatively protected by the cell wall. As a consequence, the cells studied are fixed and permeablized prior carrying out the hybridization study. Several techniques are well known in the art for premeablizing studied cells. A specific method is given hereinafter. In addition, some workers treat the studied cells with a protease such as trypsin, chymotrypsin and proteinase K. The particular method of permeabilization utilized in an _in situ_ hybridization method is typically a matter of choice that can affect quantititive determinations but usually does not affect qualitative determinations.

The phrase "hybridization conditions" when used with a maintenance time period is utilized herein to mean that a probe and the biological sample to be assayed are admixed under conditions that provide permeablized cells, single-stranded annealable molecules, as appropriate, and that the admixture so formed is maintained under temperature conditions and for a time period sufficient for the probe and any complementary polynucleotide present in the sample to anneal and form a double-stranded molecule. Such time and temperature conditions are well known in the art, and are exemplified herein in the _in situ_ hybridization studies discussed hereinafter.

The term "vector" is used herein as being synonymous for a cloning vehicle, and includes a plasmid, cosmid, phage DNA or other polynucleotide sequences that are able to replicate in a replication medium such as a bacteria or yeast. A vector is characterized by containing one or a small number of restriction endonuclease recognition sites at which the polynucleotide sequence can be cut in a determinable manner without loss of an essential biolojical function of the vector, e.g., replication, production of coat proteins or loss of promoter or binding sites, and by containing a marker suitable for use in the identification of transformed cells, e.g., tetracycline resistance or ampicillin resistance.

Plasmids, which are extrachromosomal, cyclic polynucleotides are utilized as exemplary vectors herein. A plasmid vector free from an HCMV-derived polynucleotide sequence is sually referred to herein as a "plasmid", whereas a plasmid vector containing an HCMV-derived sequence is usually referred to herein as a "recombinant plasmid".

The following abbreviations and symbols are used herein.

| bp | - | base pair(s) |
| kb | - | 1000 bp |
| K | - | kilodalton(s) |
| $M_r$ | - | apparent relative molecular mass |
| DNA | - | deoxyribonucleic acid |
| cDNA | - | complementary DNA |
| RNA | - | ribonucleic acid |
| mRNA | - | messenger RNA |
| replicon | - | the unit that controls individual acts of replication; it has an origin at which replication is initiated and it may have a terminus at which replication stops. |

When used in a context describing or depicting nucleotide sequences, the purine or

-26-

pyrimidine bases forming the nucleotide sequence are depicted as follows:

A    ·· ·   deoxyadenyl

G    -    deoxyguanyl

C    -    deoxycytosyl

T    -    deoxythymidyl


## II. PLASMID VECTORS

Human cytomegalovirus (HCMV) infections can be transferred by blood and have been shown to affect lymphocyte functions as discussed before. Infection of lymphocytes with CMV appears to induce an abortive or latent infection [Rice et al., Proc. Natl. Acad. Sci. USA, 81, 6134 (1984); and Schrier et al., Science, 230, 1048 (1985)], the nature of which is not yet defined. According to the present invention, an in situ hybridization method (discussed hereinafter) provides a sensitive and precise means for detecting an abortive CMV infection in human peripheral mononuclear cells (PBM) such as lymphocytes. Use of the method permits definition of the state of the virus and aids in the clinical evaluation of individuals at risk from overt CMV infection. Specifically, the transcription of HCMV RNAs (mRNAs) known to be produced at different stages of viral replication can now be determined.

In particular, to detect viral nucleic acid in latently (or abortively) infected cells, cloned viral fragments were selected as probes from regions of the HCMV genome that are abundantly transcribed in these cells during an abortive infection. The terms "latent state", "latent infection", "nonpermissive infection" and "abortive infection", as used herein in various grammatical forms, mean that the virus cannot be detected in tissues or secretions by

-27-

conventional cell-culture assays but nevertheless persists in a non-replicating state or at an undetectable, possibly intermittent, level of replication.

The majority of HCMV nonpermissively infected cells demonstrate a block in the expression of late viral genes. Rice et al., <u>Proc. Natl. Acad. Sci. USA,</u> 81, 6134 (1984); DeMarchi, J. M., <u>Virology,</u> 129, 287 (1983) and LaFemina et al., <u>J. Gen. Virol.,</u> 64, 373 (1983). Thus, recombinant HCMV probes were developed that contain coding regions for early genes, as is discussed hereinafter.

The process of preparing recombinant plasmids generally comprises cleaving an isolated vector plasmid at one or more specific sites by means of a restriction endonuclease, for example, <u>Eco</u>RI, <u>Pst</u>I, <u>Bam</u>HI, <u>Bgl</u>II and the like. The plasmid, which is a circular DNA molecule, is thus converted into a linear DNA molecule by the enzyme that cuts the two DNA strands at a specific site. Other non-vector DNA is similarly cleaved with the same or a similar enzyme. The linear vector or portions thereof and linear non-vector DNAs can be covalently linked to form a single circle of single-, double- or part single/part double-stranded DNA by their admixture and reaction with a second enzyme known as a polynucleotide ligase, as is well known.

Initially, cloned fragments from the major immediate-early region of HCMV (<u>Hind</u>III E fragment) were investigated herein using the above recombinant DNA technology. The cloning of the <u>Hind</u>III E fragment of HCMV strain AD169 has been previously described. [Nelson et al., <u>J. Virol.,</u> 43, 83 (1983] and is referred to as plasmid pJN201. However, the concept of using the cloned fragment as a portion of

-28-

a DNA probe for a latent or abortive infection PBM was not discussed therein.

The HindIII E DNA fragment of HCMV AD169, for example, contains about 21.5 kb [Fleckenstein et al., Gene, 18, 39 (1982)] and about 90 percent of the coding region for transcription of immediate-early (IE) RNAs [Wathen et al., J. Virol., 41, 462 (1982) and McDonough et al., Virology, 125, 31 (1983)]. Four abundantly transcribed IE RNAs (1.9 kb, 2.2 kb, 2.3 kb and 5.0 kb) and two minor transcripts (4.0 kb and 7.0 kb) are produced in this region. Jahn et al., J. Virol., 49, 363 (1984). (See Figure 2).

A subfragment of the HindIII E DNA fragment, known in the art as the EcoRI J fragment with a size of approximately 10 kb, was spliced into the vector pACYC184 (ATCC 37033) in the EcoRI site of the chloramphenicol resistance gene, and the resulting plasmid vector denominated EcoRI J-5018 was cloned in E. coli. As shown in Figure 3, this EcoRI J fragment has also been spliced into the EcoRI site of the plasmid vector pUC18 to form the plasmid denominated EcoRI J-pUC18 that provides better yields of recombinant plasmid following growth (cloning) in bacteria such as E. coli as the replication medium to prepare quantities of plasmid DNA useful a probes.

The resulting cloned plasmids or their EcoRI J fragments when linked to an indicating means are useful in the screening of infected cell DNA and RNA for a number of reasons. In particular, the EcoRI J fragment does not contain human repetitive sequences that have recently been mapped in the AD169 genome. Therefore, this fragment does not provide false positive results as can use of other genomic sequences. Ruger et al., J. Gen. Virol., 65, 1351 (1984) and McDougall et al., J. Invest. Derma., 83, 725 (1984).

In addition, the EcoRI J fragment contains the coding region for the 1.9 kb major IE RNA. This mRNA is the most abundant transcript in t..e IE region and codes for the major IE protein having an $M_r$ of about 72,000 dalton (72 K) [as reported by Stinski et al., J. Virol., 46, 1 (1983], detected by the monoclonal antibody L14. Since, as described by Huang et al., Yale J. Biol. Med., 49, 29 (1976), the various strains of HCMV share 80 percent homology by reassociation kinetics (most of the heterogeneity existing in the repeat regions), a probe that includes a nucleotide sequence coding for the 72 K protein is preferred for screening tissues for HCMV sequences.

Moreover, as described hereinafter, the EcoRI J fragment DNA does not cross hybridize with other herpes virus DNA (e.g., HSV-1, HSV-2 or EBV). The EcoRI J fragment also contains the coding sequence for the 2.3 kb IE RNA and part of the coding sequences for the two minor transcripts (7.0 kb and 4.0 kb). Jahn et al., J. Virol., 49, 363 (1984).

As shown in Figure 3, to increase the sensitivity, smaller, more specific probes were developed from sequences coding for the 1.9 kb major IE RNA. As previously mentioned, the 1.9 kb major IE RNA is a spliced transcript that contains four exons of 1341, 185, 88 and 121 nucleotides, respectively. Thus, bases present in the genome are not present in the mRNA that is translated into protein. Stenberg et al., J. Virol., 49, 190 (1984). Three restriction endonuclease fragments have been subcloned from the EcoRI J fragment to increase the sensitivity in detecting this 1.9 kb IE mRNA.

A restriction endonuclease map depicting the coding region for this transcript is shown in Figure 4. A 1.8 kb PstI fragment (PstI m) from the EcoRI J fragment of HCMV was ligated into the polylinker of another plasmid vector pUC18 to form the plasmid vector denominated PstI m-pUC18. The PstI m fragment contains the coding sequence for one of the exons located at the 5'-end of the gene. This restriction fragment also contains a promotor region for the gene and an enhancer element that is as efficient as the enhancers of SV40 and polyoma.

Another clone also illustrated in Figure 4, denominated 1.9 kb BglII-BamHI, was prepared from the 3'-end of the 1.9 kb major IE gene. This approximately 475-base pair fragment was ligated into the polylinker of yet another plasmid vector pUC18 to form the plasmid vector denominated 1.9 BglII-BamHI-pUC18. That plasmid vector contains part of the coding sequence for the 1341-base exon of the transcript.

In addition, this 475 bp BglII-BamHI fragment can be cloned into the pSP65 Riboprobe[TM] vector. Green et al., Cell, 32, 681 (1984). This recombinant plasmid permits the in vitro synthesis of RNA complementary to the 1.9 kb IE transcript.

Another clone also illustrated in Figure 3, denominated pIEBamHI-RI, was prepared from the 5'-end of the EcoRI J fragment relative to the 1.9 kb major IE gene. This approximately 4.2 kb fragment was ligated into the polylinker of another plasmid vector pUC19 at the vector's BamHI and EcoRI sites to form the plasmid pIEBamHI-RI.

Figure 3 illustrates the cloning of these fragments. Using the clones containing these three fragments; i.e., plasmid vectors containing the approximately 1.8 kb PstI m, the about 475 base BglII-BamHI and the about 4.2 kb BamHI-EcoRI fragments, probes can be prepared that have sufficiently high specific activities to detect the 1.9 kb major IE RNA that is transcribed during nonpermissive, latent infection.

In addition, as shown in Figure 5, the present invention also includes an HCMV DNA probe for determining whether late genes are expressed in human body samples such as tissue biopsies and peripheral blood lymphocytes. The late gene probe is preferably utilized in conjunction with one of the before-discussed IE probes to detect infection at stages in addition to IE latent infection, although a late gene probe of the invention can also be utilized alone as to ascertain infection at stages later than during IE transcription and in the absence of recoverable virus.

An approximately 6.6 kb BamHI fragment (denominated BamHI R) from HCMV strain AD169 was cloned into the BamHI site of the polylinker of plasmid vector pUC19 to form the plasmid denominated pCMBamR. This part of the HCMV genome is transcribed during the late phase of infection, and is inactive during the early phase of infection. DeMarchi, Virology, 114, 23 (1981); Wathen et al., J. Virol., 41, 462 (1982) and McDonough et al., Virology, 125, 31 (1983). Moreover, RNA hybrid-selection studies demonstrate that the BamHI R fragment codes for the 61 K and 71 K late polypeptides Nowak et al., Virology, 134, 91 (1984).

Utilizing these clones, HCMV DNA was detected by in situ hybridization of permissively infected cells in the brains of patients with the acquired immune deficiency syndrome (AIDS) in the kidneys of renal transplant patients and RNA in peripheral blood mononuclear cells from natural,

-32-

abortive infection Schrier et al., Science, 230, 1048 (Nov., 1985), and discussed hereinafter.

Thus, one aspect of the invention constitutes a recombinant novel plasmid vector that consists essentially of an immediate-early DNA sequence selected from the group consisting of (1) the approximately 10 kb EcoRI J fragment of the HindIII E fragment of the HCMV genome, (2) the approximately 1.8 kb PstI m fragement of the EcoRI J fragement of the HCMV genome, (3) the approximately 4.2 kb BamHI-EcoRI fragment of the EcoRI J fragment of the HCMV genome, (4) the approximately 475 bp BamHI-BglII fragment of the EcoRI J fragment of the HCMV genome, (5) the EcoRI-PstI fragment containing that BamHI-BglII approximately 475 bp fragment, and (6) the approximately 6.6 kb BamHI R fragment from the A-D EcoRI fragment of the HCMV genome operatively ligated into an appropriate plasmid vector.

The yectors used herein in which the novel polynucleotide sequences are ligated and replicated are particularly preferred vectors. The particular vectors used herein for ligation and replication are available in commerce. For example, cosmid pHC79, and plasmid vectors pUC18 and pUC19 are available from Bethesda Research Laboratories, Life Technologies, Inc., Gaithersberg, MD; Riboprobe[TM] plasmid vector pSP65 is available from Promega Biotec, Madison, WI; and plasmid vector pACYC184 is availablea from the American Type Culture Collection (ATCC), Rockville, MD. Restriction maps of each of those vectors are published and are well known in the art.

A wide range of additionally useful vectors are commercially available as are appropriate host replication media. Exemplary vectors, both plasmids and bacteriophages and hosts are available from the American Type Culture Collection of Rockville, MD, and are listed in its CATALOGUE OF BACTERIA, PHAGES AND and rDNA VECTORS, sixteenth ed., 1985. In addition, useful plasmids, cosmids and cloning vectors are listed as being available in catalogues from Boehringer Mannheim Biochemicals of Indianapolis, IN; Bethesda Research Laboratories, Inc. of Gaethersberg, MD, and Pharmacia, Inc. of Piscataway, NJ.

Appropriate other vectors into which one of the above DNA sequences can be ligated and thereafter replicated contain one or more sites of cleavage by restriction enzymes so that a desired DNA sequence can be operatively ligated therein. Such vectors can be replicated in a replication medium such as a unicellular organism like E. coli, S. cerevisiae or the like. The vector contains a replicon that is compatible with the replication medium so that the ligated DNA sequence can be propagated by the vector.

For that purpose, one or more transcriptional promoters and enhancers are operationally linked to the DNA sequence adjacent to the 5'-terminus thereof. The promoter can be endogenous to the vector or exogenous to the vector as is the enhancer sequence present in the PstI m fragment discussed before, which when that fragment was ligated into plasmid vector pUC 18 provided the resulting plasmid vector with a promoter and enhancer.

III.   NUCLEIC ACID PROBES

A nucleic acid probe; i.e., an indicating means operatively linked to a polynucleotide sequence complementary to a polynucleotide sequence to be assayed for; i.e., a sequence that codes for an immediate-early protein or for a late protein of HCMV, consitutes another aspect of this invention. The polynucleic acid of the probe can be DNA or RNA as described before, but for ease of description, DNA-containing probes are utilized for illustration.

A nucleic acid probe of this invention comprises a plasmid or polynucleic acid that contains an operatively linked indicating means. That indicating means is linked to a polynucleotide sequence of the recombinant plasmid itself or of the HCMV-related polynucleotide portion of the recombinant plasmid. Where that plasmid is excised for use as the probe that consists essentially of (1) the EcoRI J fragment of the HindIII E fragment of the genome of HCMV that contains about 10 kb, (2) the PstI m fragment of that EcoRI J fragment that contains about 1.8 kb; (3) the approximately 4.2 kb BamHI-EcoRI fragment of the EcoRI J fragment of the HCMV genome; (4) the BamHI-BglII fragment of that J fragment that contains about 475 bp; (5) the EcoRI-PstI fragment containing the BamHI-BglII fragment flanked by remnants of the pUC18 polylinker site; and (6) the BamHI R fragment of about 6.6 kb from the A-D EcoRI fragment of HCMV.

The linked indicating means is preferably inserted into a desired DNA sequence by the so-called "nick translation" method of Rigby et al., J. Mol. Biol., 113, 237 (1977), as is well known. In that method, the DNA sequence is partially broken (nicked) at one or more places and then remade using E. coli DNA polymerase I along with one or more suitably indicator means-linked labeled nucleotides.

In the specific examples that follow, the radiolabeled nucleotides were utilized as the indicating means. Those radiolabeled nucleotides

-35-

were 5'-[alpha-$^{35}$S-thio]deoxyadenosine triphosphate and 5'-[alpha-$^{35}$S-thio]deoxycytosine triphosphate.

Another convenient indicating means for the probes of this invention utilizes a biotinylated nucleotide and an enzyme such as horseradish peroxidase (HRP) linked to avidin. A commercially available biotin/avidin system is that sold under the trademark Detek I-hrp by Enzo Biochem, Inc. of New York, NY and utilizes 2'-deoxyuridine triphosphate 5-allylamine-biotin in conjunction with HRP linked to Strepavidin. The use of the above and other exemplary biotinylated uridine triphosphate derivatives in DNA probes is discussed in Brigatie et al., Virology, 126, 32 (1983).

Where the probe used is an RNA molecule, the indicating means for the probes is incorporated into the molecule during in vitro transcription. Exemplary of such in vitro transcription systems is the Riboprobe$^{TM}$ vector pSP65 from Promega Biotec (Madison, WI).

In yet another system, radioactive phosphorus, $^{32}$P, can be utilized as the indicating means. Here, the radiolabel is incorporated into the alpha phosphate of a nucleotide triphospate that is included in the replication medium when a plasmid to be made into a DNA probe is cloned. A radioactive carbon atom, $^{14}$C, or hydrogen atom, $^{3}$H, can also be incorporated into a desired nucleotide triphosphate that is admixed into the replication medium.

Following nick-translation, in vitro transcription or replication in an appropriate radionucleotide-containing medium, the resulting probe is separated from unincorporated nucleotides, medium and cellular debris. Typical means of

-36-

separation utilize spin columns and passage through sizing columns as are also utilized for plasmid recovery.

While a probe made of DNA can hybridize to DNA in a cell, the amount of DNA present of a particular gene for which hybridization is sought is typically so small that it is difficult to detect, especially if fewer than all of the cells present contain the DNA of interest as is the case with HCMV infection. However, in the case of HCMV, the mRNA that codes for the IE-72 K protein is particularly abundant even in abortively infected cells. The results discussed hereinafter illustrate binding (annealing) of a DNA probe described herein with a HCMV-derived RNA in HCMV infected PBMS, kidney and brain. Similarly, the probe that corresponds to mRNAs abundantly translated late in the infection of permissively infected cells also bound to RNA in those cells.

IV.  DIAGNOSTIC METHODS AND SYSTEMS

A method for detecting latent human cytomegalovirus in a human cellular body sample such as PBMs like lymphocytes according to this invention comprises the steps of admixing a sample containing fixed, permeabilized human cells such as PBMs with a recombinant HCMV-derived immediate-early probe of this invention; maintaining the admixture under hybridization conditions for a time period sufficient for annealing of the probe to complementary DNA and RNA (polynucleic acids) present in the sample to occur to form a complex; separating the complexed probe and its complementary sequence from any uncomplexed probe; and assaying for the presence of a signal from the indicating means.

-37-

Similar steps are followed using a late gene probe of this invention for the situation in which the transcription of late genes is to be detected. Both probes can be separately utilized on parallel samples such as tissue samples where the state of the infection is to be assayed.

Sample sizes for such in situ hybridization are well known in the art and are exemplified hereinafter. For example, where microscope slides are used as a substrate on which PBM to be assayed are fixed, about 20,000 to about 30,000 cells per slide are typically used. Alternatively, where tissues such as kidney or brain are to be assayed, thin-sections are prepared as described in the Materials and Methods Section.

The amount of probe utilized and the period of maintenance for hybridization are largely a function of the indicating means, and its amount operatively linked in the probe. For example, Angerer et al., Nuc. Acids Res., 9, 2819 (1981) reported use of 0.1-1.0 nanomoles of $^3$H poly U and $^3$H poly C at 1.4 Ci/millimole and 2.0 Ci/millimole, respectively, with a one hour hybridization time. On the other hand, Brahic et al., Proc. Natl. Acad. Sci. USA, 75, 6125 (1978) reported use of 2 nanograms per slide of [$^3$H]cDNA having a specific activity of $2 \times 10^8$ cpm/microgram to detect 10 to 20 copies of viral RNA for a 48-60 hour hybridization time. Brigati et al., Virology, 126, 32 (1983) reported use of 10-20 microliter aliquots containing 20 micrograms/milliliter of biotinylated DNA probe for viral DNA in cells with a 12-16 hour hybridization time.

The assay for the presence of the indicating means occurs after separating any unreacted probe

-38-

from that which has reacted and complexed with a complementary sequence. That separation is typically carried out by washing or rinsing with water or another appropriate liquid.

The assay itself depends upon the indicating means linked to the probe. Where the indicating means is a radiolabel, autoradiography or scintillation counting techniques are the tools of choice. Where the label is an enzyme that is used to form a colored product as in some biotinylated probes, color photomicroscopy is utilized to provide a permanent record of the assay. Fluorescent indicators can also be linked to strepavidin that is used with a biotinylated probe to provide the assay in conjunction with fluorescence photomicroscropy as reported by Brigati et al., Virology, 126, 32 (1983).

Another aspect of this invention is a diagnostic assay system typically in kit form for detecting the presence of HCMV in a human cellular body sample such as PBMs, kidney or brain. For PBMs, such an assay system is particularly useful in screening blood products for HCMV prior to transfusion.

The system comprises at least one container or package, and includes as an active ingredient, an amount of a recombinant immediate-early HCMV-derived probe of this invention sufficient to react with and anneal to complementary nucleic acid sequences in the sample.

The above-described diagnostic system is particularly useful in detecting the presence of a latent HCMV infection in blood products. Where the state of an HCMV infection is desired to be assayed, a second diagnostic system is useful.

That second diagnostic system comprises at least two containers or packages, and includes as active ingredients (a) the active ingredients of the above-discussed diagnostic system and (b) an amount of a recombinant late HCMV-derived probe of this invention sufficient to react with and anneal to complementary nucleic acid sequences in the sample.

The indicating means for signalling the reaction between said recombinant plasmid or DNA probe and the complementary nucleic acid sequences in the sample can include radioisotopes such as $^{32}P$, $^{14}C$, $^{3}H$ and $^{35}S$. Another useful indicating means is biotin, as already noted, that can produce a biotin-labeled probe that is stable for up to two years when stored at -20 degrees C. Biotin-containing probes are typically used with avidin-containing amplifying and visualizing reagents, as also already noted. Where an indicating means that requires one or more additional reagents such as HRP is the linked indicating means, those additional reagents can also be included in the system.

V. RESULTS

A. HCMV Infection in Peripheral
Blood Mononuclear Cells

The results obtained using a before-described probe and method using lymphocytes as the cellular human body sample indicate that peripheral blood mononuclear cells from asymptomatic, HCMV-seropositive and T cell proliferation-positive donors synthesize polynucleic acid sequences that hybridize to: a) a portion of the genome heavily transcribed during immediate-early periods of infection and also to b) fragments from areas transcribed late in the replicative cycle. These probes do not detect sequences in uninfected or HSV-infected fibroblasts. The proportion of blood cells hybridizing varied from individual to individual (1/50 - 1/200), and since the signal was reduced by hydroxide ion hydrolysis and RNAase, it can be concluded that RNAs are the polynucleic acid sequences that are synthesized by the individuals whose PBM were studied and are the polynucleic acid sequences being detected by the probes.

These observations imply a relatively active, though latent and nonproductive, state for the virus in natural infection and indicate a role for a lymphocyte as a site for maintenance of HCMV infection in the host. The observations are discussed below.

Examination of PBMs from eight individuals who were seropositive for HCMV and twelve who were seronegative gave the following results. Strong hybridization with the pUC18-EcoRI J probe was found in 2 percent of the PBMs (dots containing $10^4$ cells were scanned) from two of the eight asymptomatic seropositive individuals (Figures 6, Panels 1 and 5). This level of hybridizaton was consistent over several months in six to eight different samples for each individual. Neither the vector plasmids pUC18 or pUC19 (Figure 6, Panel 8), nor an HSV-2 probe (Bgl II fragment C) bound to these cells. Hybridization with the EcoRI J-containing probe was found in 0.03 to 1 percent of the PBMs from the other six seropositive individuals.

Cells from 11 of the 12 seronegative individuals showed no hybridization to the above HCMV probe, but 0.1 percent of the cells from the 12th individual hybridized to the probe. The antibody may have been absent in this individual because viral

-41-

protein was insufficient to generate a response or because the individual did not respond to this virus. A similar lack of detectable antibody has been observed in a subset of patients infected with human T cell lymphotropic virus, type III (as determined by culture of virus) Salahuddin et al., Lancet 1984-II, 1418 (1984).

Treatment with 0.1M NaOH (Figure 6, Panel 3) or ribonuclease (100 ug/ml, 30 minutes, 37°C) significantly reduced signal and indicated that hybridization was primarily with HCMV RNA. Heat treatment (100°C for 2 minutes) did not increase the hybridization signal in PBMs.

In contrast, similar treatment of acutely infected fibroblasts increased the signal, presumably by hybridization of the probe to denatured viral DNA. Culture of lymphocytes from hybridization-positive donors on permissive fibroblasts produced no cytopathic effects; these results are consistent with many observations that HCMV-infected lymphocytes produce few, if any, infectious virions under such circumstances.

To determine if a specific subpopulation of PBMs harbored HCMV, a fluorescence-activated cell sorter (FACS) was used to separate cells from a seropositive (2 percent PBM-positive) individual. The lymphocytes were stained with antibodies detecting the two major T cell subpopulations, OKT4 and OKT8. Although this technique provides few cells, the purity of the populations exceeds 97 percent.

Results from three such studies indicated that a higher percentage of HCMV-hybridizing cells bore the OKT4 marker (2.4 percent) than the OKT8 antigen (0.8 percent) (Figure 6, Panels 6 and 7) (in

-42-

unseparated blood, 44 percent of the cells were OKT4 and 18 percent were OKT8). This observation not only demonstrates some selectivity of HCMV infection, but confirms that lymphocytes, specifically in the PBM preparations are infected and contain HCMV RNA.

Whether HCMV RNA is present in other PBM subpopulations remains to be determined. Preliminary results indicate that monocytes also express the IE HCMV transcript.

In a further study, PBMs from normal HCMV seropositive donors were obtained for a period of one year and studied to determine whether hybridization levels using an EcoRI J fragment-containing probe were stable for a given individual or varied over time. As can be seen from Figure 9, PBMs from two donors (G and R) showed no hybridization and no change over the time period studied. PBMs from donor S remained relatively high in hybridization, whereas those from donor P began with a relatively high percentage of cells hybridizing and dropped to an undetectable level at the end of the study period. PBMs from donor B showed an increasing number of hybridizing cells.

The factors controlling these variations are currently under investigation. The unknown variation-controlling factors notwithstanding, the results discussed in this section illustrate further that seropositivity or seronegativity alone do not provide a complete description of the HCMV disease state.

The before-described probe has been also shown to detect polynucleotides in HCMV-infected fibroblasts. The probe did not detect a polynucleotide in uninfected or HSV-infected fibroblasts.

-43-

### B. Comparison of Immediate-Early and Late RNA Transcription of HCMV Genes in Kidney

Kidney biopsies were taken during the course of kidney transplantation therapy of HCMV seronegative transplant recipients. Those biopsy samples were screened for immediate-early and late HCMV RNA transcripts by in situ hybridization.

As shown in Table 1, donor kidneys before transplantation ("Pre-transplant") do not exhibit reactivitiy within any of the probes used. However, about one month after implantation ("Post-transplant") HCMV infection spread into the kidney and was detected in a differential manner depending on the probe used. Further, HCMV-infected tissue was detected in ratios of immediate-early to late that were different from the earlier pattern of detectable infection after about 2 to 3 weeks of experimental anti-HCMV therapy using the investigational drug, Foscarnet ("Post-Foscarnet").

### TABLE 1

#### In Situ Hybridization Of Kidney Biopsies With Immediate-Early And Late Probes Of HCMV[1]

| Patient | Probe[2] | Pre-Transplant | Post-Transplant | |
| --- | --- | --- | --- | --- |
| | | | Pre-Foscarnet | Post-Foscarnet |
| A | R1J | 0 | ++++ | |
| | PCM3 | 0 | +++ | [3] |
| | PUC | 0 | 0 | |
| B | R1J | 0 | +++ | +++ |
| | PCM3 | 0 | +++ | +++ |
| | PUC | 0 | 0 | 0 |

| | | | | |
|---|---|---|---|---|
| C | R1J | 0 | ++++ | + |
| | PCM3 | 0 | ++ | +++ |
| | PUC | 0 | 0 | 0 |
| D | R1J | 0 | ++ | 4 |
| | PCM3 | 0 | +++ | N.D. |
| | PUC | 0 | 0 | |
| E | R1J | 0 | + | ++ |
| | PCM3 | 0 | 0 | +++ |
| | PUC | 0 | 0 | 0 |
| F | R1J | 0 | ++ | |
| | PCM3 | 0 | ++ | --- |
| | PUC | 0 | 0 | |
| G | R1J | 0 | ++ | + |
| | PCM3 | 0 | +++ | +++ |
| | PUC | 0 | 0 | 0 |

[1]Hybridization intensity scale = 0 (no signal) to ++++ (most intense), measured by numbers of infectious centers in a given microscopic field of sample. Tissue samples were obtained from patients and prepared as described in the Materials and Methods Section.

[2]Probes: RIJ = EcoRI J fragment from the HCMV immediate-early gene; pCM3 = EcoRI A-D fragments from the HCMV late gene; pUC = pUC18 plasmid control.

[3] "-" indicates no sample submitted for testing from this patient.

[4]"N.D." indicates sample not tested, thus no data.

Over time, the pattern of immediate-early and late probe reactivity can shift depending upon the patient. Also, in particular cases, infected kidneys exhibit a stronger reactivity with immediate-early probe over late probe, whereas in other cases-the opposite is observed.

This embodiment of detecting HCMV by a combination of immediate-early and late probe together is suggested by the above data in which a few cases show hybridization to one probe but little or no hybridization with the other. See for example patient E "Pre-Foscarnet", or patients C and G "post-Foscarnet".

Examples of the degree of hybridization and the extent of HCMV infection in these kidney biopsies is shown in Figure 7. Thin sections prepared from HCMV infected patients at the "Pre-Foscarnet" stage were _in situ_ hybridized to either HCMV immediate-early (Frame A), late (Frame B) or non-HCMV control probe (Frame C). Frame A contains the highest degree of hybridization (++++), Frame B contains less (+++) and Frame C has none.

These data indicate that although probes from either immediate-early or late HCMV genes are effective in detecting HCMV RNA transcripts, a combined modality utilizing both immediate-early and late HCMV probes is preferred where the objective is to detect HCMV infection rather than to distinguish permissive from latent HCMV infection.

C. HCMV Infections in Brain is Detectable by In Situ Hybridization

Thin sections of paraffin-embedded brain tissue were analysed by _in situ_ hybridization using the immediate-early HCMV gene probe, EcoRI J, as described hereinafter. As shown in Figure 8 brain

-46-

tissue analysed in this manner showed numerous cells with dense precipitates of silver grains. These cells were most concentrated rimming the periventricular area, but were also found deeper in the brain. Control hybridizations in which the probe recognized HSV genes were uniformly negative, as were hybridizations using the CMV probe on sections from normal brains.

VI. MATERIALS AND METHODS

A. HCMV-Infected Cultures and Tissues

CMV strain AD 169 (ATCC VR-538) was maintained in Flow 5000 fibroblasts (Flow Laboratories; McLean, VA) as described by Nelson et al., J. Virol. 43, 83 (1982), incorporated herein by reference. For infection of fibroblasts, infected fibroblasts were sonicated, the cellular debris was removed by centrifugation, and the liquid phase containing the virus was added to uninfected cultures at a multiplicity of infection (MOI) of approximately 3:1. Infected and uninfected fibroblasts were used as controls for in situ hybridization and were processed in a manner similar to the lymphocytes. Human peripheral blood mononuclear cells (PBMs) were obtained from normal seropositive and seronegative donors, and were isolated on Ficol-Hypaque gradients.

Seropositivity was determined by an enzyme-linked immunosorbent assay (ELISA), with desiccated infected fibroblasts used as the antigen as discussed in Rice et al., J. Infect. Dis., 147, 1055 (1983). Serum that gave twice the response (as measured in optical density units) on HCMV-infected versus uninfected fibroblasts was defined as seropositive for CMV. T cell proliferation to HCMV (2x background) after 5 days of culture correlated with results from the ELISA. PBMs so obtained can be used directly and plated on slides for in situ hybridization, or they can be cultured for later use, or further separated by flourescence-activated cell sorting (FACS) as described below.

Separation of PBM cell populations by FACS into subpopulations was conducted using biotinylated OKT4 or OKT8 antibody (Beckton-Dickinson, Mountain View, CA). Briefly, $2x10^7$ PBMs were maintained for approximately 40 minutes at zero degrees C in antibody diluted 1:10 in phosphate-buffered saline (PBS) at about $1x10^7$ cells/ml. Cells were washed twice in PBS prechilled to zero degrees C, and were thereafter incubated for 30 minutes in flourescent isothiocyanate-coupled avidin (Becton-Dickinson) at a dilution of 1:50 in PBS. Cells were then washed in PBS, resuspended in 3 ml of PBS and sorted at a FACS window setting set to select only the very brightly staining cells (Oldstone et al., Virology 127, 426, 1983). Cells so obtained by FACS were then used for in situ hybridization in a manner similar to the lymphocytes.

Formalin fixed and paraffin-embedded brain tissue samples were taken at autopsy from a patient afflicted with AIDS, and were provided by Dr. C.A. Wiley (Dept. of Pathology, University of California, San Diego, CA). Formalin fixed and paraffin-embedded kidney biopsies were taken from patients undergoing kidney transplantation therapy, and were provided by Dr. Lars Olding (Department of Pathology, Karolinska Institute, Stockholm, Sweden). Transplantation therapy included treatment after kidney implantation with the investigational drug phosphonoformate (Foscarnet).

B. Hybridization

In situ hybridization was performed according to modified methods of Brahic et al., Proc. Natl. Acad. Sci. USA, 75, 6125 (1978), and Angerer et al., Nucleic Acids Res., 9, 2819 (1981).

Briefly, human peripheral blood mononuclear cells were obtained from normal seropositive and seronegative donors, and were isolated on Ficol-Hypaque gradients.

Cells to be used directly were washed three times in PBS, adjusted to $10 \times 10^6$ cells per milliliter, and 5 to 10 microliter quantities were plated on acid-cleaned polylysine-coated slides prepared as described hereinafter. Slides were then maintained in air for a time period sufficient for the plated cells to dry. Air-dried cells on slides were then fixed for 15 minutes with periodate-lysine-paraformaldehyde (PLP) as described in McLean et al., J. Histochem.·Cytochem., 22, 1077 (1974), rinsed twice in PBS, dehydrated by washings with graded ratios of water to ethanol up to 95 percent ethanol, and were stored at 4 degrees C.

Glass microscope slides were acid-cleaned and polylysine-coated for use as substrate to support samples for in situ hybridization. Briefly, slides were soaked 4 to 24 hours in chromic acid in stainless steel staining racks. After cleaning, the slides were rinsed for 4 hours with tap water and then washed three times in deionized water. Cleaned and rinsed slides were soaked for 30 minutes in 0.01% polylysine (Sigma Chemical Co., St. Louis, MO #P7886), rinsed three times in distilled water, dried at 37 degrees C and baked at 80 degrees C for 1 hour.

-49-

Tissue sections of paraffin-embedded kidney or brain tissue were prepared for in situ hybridization by slicing the sample at about 5 to 10 micrometer thicknesses. The resulting thin sections were floated on 45 degrees C distilled water containing 1.0 percent Elmer's white glue. The floating specimins were picked up using the polylysine-coated slides and air dried overnight.

As a further preparation in situ hybridization, samples are hydrate and permeabilized. Tissue sections were deparaffinized by washing in xylene three times for 10 minutes each. All samples whether they are fixed PBMs, fibroblasts or sections were then rehydrated through washings in graded ratios of ethanol to water at about 1 minute per wash in the following series: 100 percent, 95 percent, 70 percent, 50 percent and 30 percent, wherein all solutions except 100 percent ethanol contained 0.33 M ammonium acetate.

Samples were prepared for permeabilization by two washes in distilled water for about 1 minute each, one wash in 0.2 M HCl for about 10 minutes, two more washes in distilled water as above, one wash in one percent Triton X-100 [polyoxyethylene (9) octyl phenyl other] for 1.5 minutes and a final 2 rinses in distilled water for 1 minute each, wherein all rinses above were conducted at room temperature.

Fibroblast and thin sections, but not the PBM samples, were then treated with proteinase K (1 microgram/ml in 20 mM Tris HCl and 2 mM $CaCl_2$) for 20 minutes at 37 degrees C, and then soaked for 4 minutes in PLP at room temperature. All samples were then washed in PBS-glycine (2 grams/liter of glycine), washed once in PBS, and gradually dehydrated to about 100 percent ethanol at which time the samples are ready for hybridization probe mix to be added.

The hybridization probe mix consisted of 50 percent deionized formamide, 5X Denhardt's solution, 5X hybridization salts (0.9 M NaCl, 50 mM $NaH_2PO_4$ and 5mM EDTA), 10 percent Dextran sulfate, heparin (30 Units/ml), 0.1 percent Triton X-100, salmon sperm DNA (500 micrograms/ml) and BHK cell RNA (250 micrograms/ml). $^{35}$S-labeled probes were added to a concentration of 0.25 to 0.5 micrograms/ml (about $1 \times 10^8$ cpm/microgram), and the entire mixture was boiled for 2 minutes and then chilled on ice. Dithiothreitol was thereafter added to a concentration of 10 mM.

The solution was placed on the above-prepared cells, covered with sheets or cover slips of Gel-bond (FMC Corporation, Rockland, ME), and sealed with rubber cement. Hybridization was carried out by incubation (admixture and maintenance) of the cell-probe admixture for 18 hours at 37 degrees C in a humidified chamber.

Following incubation, the cover slips were removed and the slides were washed with: 2X SSC (0.3 M NaCl, 0.03 M sodium citrate) for 30 minutes at 21 degrees C, 30 minutes with 1X SSC at 21 degrees C, 10 minutes with 0.1X SSC at 60 degrees C, 20 minutes with 0.1X SSC at 37 degrees C, and 5 minutes with 2X SSC. The cells were then dehydrated using ethanol and dried. Slides were dipped in Kodak NTB-2 emulsion, exposed for 3-4 days at 4 degrees C, and then developed with Kodak D19 developer and standard fixer. Where indicated, samples were then counterstained with hematoxylin.

### C.  Plasmids

Plasmids were grown in either the host (ATCC 33694) bacterium E. coli HB101 for pHC79 or E. coli JM83 (ATCC 35607). Plasmids pUC18 and pUC19 were gifts from Dr. R. Gelinas (Fred Hutchinson Cancer Research Center, Seattle, WA). Plasmid pACYC184 was obtained from the American Type Culture Collection (ATCC, Bethesda, MD, #37033), and Riboprobe™ vector pSP65 was obtained from Promega Biotec, Madison, WI. Cells containing plasmids were cultured in Luria broth [1.0 percent tryptone broth (Difco Laboratories), 0.5 percent yeast extract (Difco) and 1.0 percent sodium chloride and were amplified during logarithmic growth by the addition of chloramphenicol (170 micrograms per ml) per milliliter of Luria broth (LB).

Culture medium routinely included antibiotics to maintain selective pressure on the plasmids present in their host bacterium, and the choice of antiboiotics depends upon the plasmid being grown. E. coli containing plasmid vectors pUC18, pUC19 and pSP65, were grown in the aforementioned L-Broth containing ampicillin at 100 mg/ml, and those containing pACYC184 were similarily grown except that 25 mg/ml tetracycline was used.

Plasmid DNA was prepared by a modification of the alkaline lysis method [Birnboim et al., Nucleic Acids Res., 7, 1513 (1979)], and was further purified by equilibrium centrifugation through cesium chloride gradients containing ethidium bromide.

Ligation of the viral fragments and transformation of the bacteria with ligated plasmid was performed as described by Jahn et al., J. Virol., 49, 363 (1984), and Nelson et al., J. Virol., 43, 83 (1983). Each viral DNA insert was identified after

-52-

endonuclease digestion by electrophoretic comigration with digests of cosmid-cloned viral DNA according to the method reported by Fleckenstein et al., Gene, 18, 39 (1982), and was verified by Southern blot hybridizations to different DNA fragments of virion DNA according to the method of Southern, E. M., J. Mol. Biol., 98, 503 (1975).

1) Cloning of the EcoRI J Fragment

Referring to Figure 3, the EcoRI J fragment was subcloned from the HindIII E fragment of HCMV strain AD169 by digesting the DNA of plasmid vector pJN201 (discussed below) with the restriction endonuclease EcoRI (New England Biolabs, Inc., Beverly, ME), isolating the DNA from low melting agarose (Bethesda Research Laboratories, Life Technologies, Inc., Gaithersberg, MD; hereinafter BRL), and ligating the approximately 10 kb J fragment DNA into the EcoRI site of either the plasmid vector pACYC184 or pUC18 to form plasmid vectors EcoRI J-5018 and EcoRI J-pUC18, respectively.

Plasmid pJN201 is a construct of the HindIII E HCMV fragment ligated into plasmid pHC79 that is available from BRL, and was prepared as described in Nelson et al., J. Virology, 43, 83 (1982). The construction of the pACYC184 plasmid is described in Chang et al., J. Bacteriol., 134, 1141 (1978), which is incorporated herein by reference. That plasmid is available from the ATCC as 37033. Plasmids pUC18 and pUC19 are also available from BRL.

EcoRI is a restriction endonuclease isolated from Escherichia coli RY13 that recognizes and cleaves the following site of a nucleotide sequence: G AATTC. By convention, nucleotide sequences are written from left to right and in the direction from 5'-end to 3'-end.

-53-

Bacteria containing the pACYC184 recombinant plasmid vector EcoRI J-5018 were grown in LB media containing 25 micrograms per milliliter (micrograms/ml) tetracycline, while those bacteria containing the pUC18 recombinant vectors were grown in media containing 100 micrograms/ml ampicillin.

2) PstIm-pUC18 and 1.9 BglII-BamHI-pUC18 clones

Referring again to Figure 3, the PstI m fragment containing approximately 1.8 kb was subcloned from the EcoRI J fragment of HCMV strain AD169 by digesting the EcoRI J-pUC18 plasmid with PstI (New England Biolabs, Inc., Beverly, Maine), isolating the 1.8 kb DNA fragment denominated "m", and ligating the PstI m DNA fragment into the PstI site of further pUC18 to form the recombinant PstI m-pUC18.

PstI is a restriction endonuclease isolated from Providencia stuartii 164 that recognizes and cleaves the following site of a nucleotide sequence: CTGCA G.

The 475 bp BglII-BamHI fragment was subcloned from the EcoRI J fragment of the EcoRI J-pUC18 plasmid vector by digestion of the cloned vector DNA with endonucleases BglII and BamHI (New England Biolabs, Inc.). The 475bP fragment was isolated and ligated into the BamHI site of the polylinker of still more pUC18 to form the 1.9 BglII-BamHI-pUC18 plasmid.

BglII is a restriction endonuclease that is isolated from Bacillus globigii that recognizes and cleaves the following nucleotide sequence: A GATCT. V. Pirrotta, Nucleic Acids Res., 3, 1747 (1976).

Referring to Figure 3, the approximately 4.2 kb BamHI-EcoRI fragment was subcloned from the

-54-

EcoRI J fragment of the EcoRI J-pUC18 plasmid vector by digestion of the cloned vector DNA with endonucleases BamHI and EcoRI. The 4.2 kb fragment was isolated and ligated into the BamHI and EcoRI sites of pUC19 to form the recombinant pIEBamHI-RI.

BamHI is a restriction endonuclease isolated from Bacillus amyloliquefaciens H that recognizes and cleaves the following site of a nucleotide sequence: G GATCC. Roberts et al., Nature, 265, 82 (1977).

      3) Construction of pSP65 1.9 Bgl II-BamHI

The pSP65-1.9 BglII-BamHI plasmid is constructed by digesting the 1.9 BglII-BamHI-pUC18 plasmid with EcoRI and PstI. The resulting fragment contains the 475 bp BglII-BamHI fragment as well as relatively short sequences from the polylinker on either side of the BamHI site. The resulting fragment that contains about 515 base pairs is isolated and ligated into the EcoRI and PstI sites of Riboprobe[TM] pSP65. The resulting plasmid vector, denominated pSP65-1.9 BglII-BamHI, is linearized, and transcribed with RNA polymerase in the presence of 5'-alpha-$^{35}$S or alpha-$^{32}$P labeled ribonucleotides to form a radiolabeled RNA probe.

      4) Construction of the pCMBamR Clone

Referring to Figure 5, the BamHI R fragment of HCMV strain AD169 was subcloned from the cosmid pCM3, reported by Fleckenstein et al., Gene, 18, 39 (1982) and containing the EcoRI A plus D (A-D) fragments cloned in cosmid pHC79, by digesting the clone with BamHI. The 6.6 kb BamHI fragment was isolated, and ligated into the BamHI site of pUC19 to form the plasmid vector denominated pCMBam R. The BamHI fragment was shown by Nowak et al., Virology,

*134*, 91-104 (1984) to code for the 61K and 71K late polypeptides. (See Figure 1).

### D. Polynucleotide Probes

Polynucleotide probes of DNA were prepared from the above plasmids as follows. An appropriate plasmid vector was nick translated following the procedures of Rigby et al., *J. Mol. Biol.*, *113*, 237 (1977) by incorporating 5'-[alpha-$^{35}$S-thio]-deoxyadenosine triphosphate and 5'-[alpha-$^{35}$S-thio]deoxycytosine triphosphate (650 Ci/mM) (Amersham Corp., Arlington Heights, IL). DNA polymerase I was purchased from New England Biolabs Inc., Beverly, MA.

The resulting nick-translated DNA was separated from nucleotides by spin (Bio-Rad Laboratories, Richmond, CA) columns spun for 2 minutes and passage through Sepharex G-50 (Pharmacia Fine Chemicals, Piscataway, NJ) at 1200xg. Using the EcoRI J-pUC18 probe as exemplary, a specific activity of 1x10$^8$ cpm per microgram of DNA was obtained.

Control probes were similarly prepared from plasmid pUC18 or a plasmid containing the BglII C fragment from HSV-2 strain 333. This fragment corresponds to the HSV genome at about 0.43 to about 0.58 map units as described by Jariwalla et al., *Proc. Natl. Acad. Sci. USA*, *77*, 2279 (1980).

The foregoing is intended as illustrative of the present invention but is not limiting. Numerous variations and modifications may be effected without departing from the spirit and scope of the invention.

-56-

WHAT IS CLAIMED IS:

1. A process for preparing a recombinant HCMV-derived plasmid comprising:

(a) digesting the HindIII E fragment of the genome of an HCMV strain with restriction endonuclease EcoRI to provide fragmented linear DNA segments including an EcoRI J fragment containing about 10 kb; and

(b) ligating the EcoRI J fragment into the EcoRI site of a plasmid to provide a recombinant HCMV-derived plasmid.

2. A process for preparing a recombinant HCMV-derived plasmid comprising:

(a) providing a first recombinant HCMV-derived plasmid that contains the approximately 10 kb EcoRI J fragment of the HCMV genome;

(b) digesting the EcoRI J fragment portion of the first recombinant HCMV-derived plasmid with restriction endonuclease PstI to provide fragmented linear DNA segments including a PstI m fragment that contains approximately 1.8 kb; and

(c) ligating the PstI m fragment into the PstI site of a plasmid to provide a second recombinant HCMV-derived plasmid.

3. A process for preparing a recombinant HCMV-derived plasmid comprising:

(a) providing a first recombinant HCMV-derived plasmid that contains the approximately 10 kb EcoRI J fragment of the HCMV genome;

(b) digesting the EcoRI J fragment portion of the first recombinant HCMV-derived plasmid with restriction endonucleases BglII and BamHI to provide fragmented linear DNA segments including an about 475 bp BglII-BamHI fragment; and

(c) ligating said BglII-BamHI fragment into the BamHI site of a polylinker site-containing plasmid to provide a second recombinant HCMV-derived plasmid.

4. A process for preparing a recombinant HCMV-derived plasmid comprising:

(a) providing a first recombinant HCMV-derived plasmid that contains the approximately 475 bp BglII-BamHI fragment of the EcoRI J fragment of the HCMV genome ligated into a polylinker site of the plasmid, said polylinker site including restriction endonulease cleavage sites for BamHI, BglII, EcoRI and PstI restriction endonucleases;

(b) digesting said first recombinant plasmid with restriction enconucleases EcoRI and PstI to provide fragmented linear DNA segments including an EcoRI-PstI fragment containing said BglII-BamHI fragment; and

(c) ligating the EcoRI-PstI fragment into the EcoRI and PstI sites, respectively, of a plasmid to form a second recombinant HCMV-derived plasmid.

5. A process for preparing a recombinant HCMV-derived plasmid comprising:

(a) providing a first recombinant HCMV-derived plasmid that contains the approximately 10 kb EcoRI J fragment portion of the HCMV genome;

(b) digesting the EcoRI J fragment portion of the first recombinant HCMV-derived plasmid with restriction endonucleases BamHI and EcoRI to provide fragmented linear DNA segments including a BamHI-EcoRI fragment that contains approximately 4.2 kb; and

(c) ligating said approximately 4.2 kb BamHI-EcoRI fragment into the BamHI and EcoRI sites, respectively, of a plasmid to form a second recombinant HCMV-derived plasmid.

6. A process for preparing a recombinant HCMV-derived plasmid comprising;

(a) providing a first recombinant HCMV-derived plasmid that contains the approximately 33 kb A plus D fragments of an HCMV strain;

(b) digesting the A plus D fragment portions of said first recombinant plasmid with restriction endonuclease BamHI to provide fragmented linear DNA segments including the BamHI R fragment that contains approximately 6.6 kb; and

(c) ligating said approximately 6.6 kb BamHI R fragment into the BamHI site of a plasmid to form a second recombinant HCMV-derived plasmid.

7. A recombinant HCMV-derived plasmid that contains an HCMV-derived sequence consisting essentially of a sequence selected from the group consisting of the approximately 10 kb EcoRI J fragment of the HindIII E fragment of the HCMV genome, the approximately 1.8 kb PstI fragment of the EcoRI J fragment of the HCMV genome, the approximately 4.2 kb BamHI-EcoRI fragment of the EcoRI J fragment of the HCMV genome, the approximately 475 bp BamHI-BglII fragment of the EcoRI J fragment of the HCMV genome, and the approximately 6.6 kb BamHI R fragment of the EcoRI A plus D fragment of the HCMV genome.

8. A diagnostic assay system for detecting the presence of HCMV in a human cellular body sample, said system including in at least one container as an active ingredient an amount of an indicating means-linked recombinant HCMV-derived polynucleotide probe corresponding in sequence to an immediate-early protein gene sufficient to react with and anneal to a complementary nucleic acid sequence in the sample.

9. The diagnostic system according to claim 8 wherein said probe contains the approximately 10 kb EcoRI J fragment of the HindIII E fragment.

10. The diagnostic system according to claim 8 further including a second container that includes as an active ingredient an amount of indicating means-linked recombinant HCMV-derived polynucleotide probe corresponding in sequence to a late protein gene sufficient to react with and anneal to a complementary nucleic acid sequence in the sample.

11. A method for detecting a latent human cytomegalovirus infection in a human sample of PBMs comprising the steps of:

(a) admixing a human cellular body sample with an amount of an indicating means-linked recombinant HCMV-derived polynucleotide probe corresponding in sequence to an immediate-early protein gene or to a late protein gene sufficient to react with an anneal to complementary nucleic acid sequence in said sample;

(b) maintaining said admixture under hybridization conditions for a time period sufficient for the probe to react with and anneal to complementary polynucleic acids in the cells of the sample and form a complex;

(c) separating the complex of annealed probe and complementary polynucleic acid from any uncomplexed probe; and

(d) assaying for the presence of said complex.

12. The method according to claim 11 wherein said probe corresponds to an immediate-early

-60-

protein gene and said probe consists essentially of a polynucleic acid sequence selected from the group consisting of the approximately 10 kb sequence of the EcoRI J fragment of the HCMV genome, the approximately 1.8 kb PstI m fragment of the EcoRI J fragment of the HCMV genome, the approximately 4.2 kb BamHI-EcoRI fragment of the EcoRI J fragment of the HCMV genome, and the approximately 475 bp BamHI-BglII fragment of the EcoRI J fragment of the HCMV genome.

13. The method according to claim 11 wherein said human body sample is a tissue sample, a first said tissue sample is admixed with a probe that corresponds in sequence to an immediate-early protein gene, and a parallel second said tissue sample is admixed with a probe that corresponds in sequence to a late protein gene that consists essentially of the approximately 6.6 kb BamHI R fragment from the A plus D EcoRI fragment of the HCMV genome.

14. A polynucleotide probe consisting essentially of a recombinant polynucleotide sequence of an HCMV-derived immediate-early gene or a late gene linked to an indicating means, said immediate-early gene probe consisting essentially of a sequence  selected from the group consisting of the approximately 10 kb EcoRI J fragment of the HindIII E fragment of the HCMV genome, the approximately 1.8 kb PstI m fragment of the EcoRI J fragment of the HCMV genome, the approximately 4.2 kb BamHI-EcoRI fragment of the EcoRI J fragment of the HCMV genome, and the about 475 bp BamHI-BglII fragment of the EcoRI J fragment of the HCMV genome, and wherein said late gene probe consists essentially of  the about 6.6 kb BamHI R fragment of the EcoRI A plus D fragment of the HCMV genome.

# FIGURE 1

## HCMV STRAIN AD169 DNA PROBES FOR *IN SITU* HYBRIDIZATION

# FIGURE 2

IMMEDIATE EARLY TRANSCRIPTION OF HCMV STRAIN AD169 Hind III E FRAGMENT

FIGURE 3

CONSTRUCTION OF IE PROBES FROM HCMV STRAIN AD169

0 271 201

# FIGURE 4

# FIGURE 5

## CONSTRUCTION OF LATE PROBES FROM HCMV STRAIN AD169

FIGURE 6

# FIGURE 7

A

B

C

# FIGURE 8

# FIGURE 9